# EUROPEAN PATENT APPLICATION

(11) **EP 3 398 600 A1**
(43) Date of publication of application: **07.11.2018**
(21) Application number: 17168957.3
(22) Date of filing: 02.05.2017
(51) Int. Cl.: A61K 31/455, A61K 33/06, A61K 33/10, A61K 33/24, A61K 33/26, A61K 45/06, A61P 3/06, A61P 3/12, A61K 47/38, A61K 9/16

(54) **MODIFIED RELEASE NICOTINAMIDE**

(71) Applicant: Salmon Pharma GmbH, 4002 Basel (CH)
(72) Inventor: AMMER, Richard, Am Wunderhügel 11 58638 Iserlohn (DE)
(74) Representative: Isarpatent

(57) **Abstract**

The present invention relates to a pharmaceutical preparation comprising modified release nicotinamide, as well as its use in a method of preventing and/or treating of elevated serum phosphate levels (hyperphosphatemia) and/or dyslipidemia, both particularly resulting from renal failure.

## Description

The present invention relates to a pharmaceutical preparation comprising modified release nicotinamide, as well as its use in a method of preventing and/or treating of elevated serum phosphate levels (hyperphosphatemia) and/or dyslipidemia, both particularly resulting from renal failure.

### Background

Hyperphosphatemia, defined as super-physiological levels of phosphate, is considered an independent risk factor for patients in chronic kidney disease (CKD) or chronic renal failure (CRF), and adequate therapy is still a challenge for which ca. 50% to 70% of CKD patients do not meet recommended target phosphate levels [KDIGO guideline 2009 (8); K/DOQI clinical practice guidelines 2003 (7)] according to DOPPS III [Young 2004 (1), Tentori 2008 (2)].
Kidney failure is the main cause of hyperphosphatemia. Chronic renal failure (CRF) is a progressive kidney disease; when the kidney has lost all its ability of clear the blood from extensive fluid volume, electrolytes, metabolic substances, the patients cannot survive and have to be referred to dialysis. Such a last condition is defined End-Stage Renal-Disease (ESRD). One of the most crucial electrolytes is phosphate.
CKD disrupts systemic calcium and phosphate homeostasis and affects the bone, gut, and parathyroid glands. This occurs because of decreased renal excretion of phosphate and diminished renal hydroxylation of 25-hydroxyvitamin D to calcitriol (1,25 dihydroxyvitamin D) [Levin, 2007 (3)]. Progressive kidney dysfunction results in hyperphosphatemia and calcitriol deficiency. These ultimately can result in hypocalcaemia. These abnormalities directly increase PTH levels via sensing the Calcium-Sensing Receptor (CaSR) as potent stimulus to the release of PTH. In consequence, hyperphosphatemia is also an important factor underlying hyperparathyroidism. Although the identity of the extracellular phosphate sensor is unknown, a novel phosphaturic factor, FGF23, may be regulated by phosphate and vitamin D. This may have a role in regulating parathyroid gland function in end stage renal disease (ESRD) [Saito, 2005 (4)].
Hyperphosphatemia also lowers the levels of ionized calcium and interferes with the production of 1,25-dihydroxyvitamin D, thereby resulting in increased PTH levels. Hyperphosphatemia and secondary hyperparathyroidism with abnormalities in serum phosphate and calcium levels are associated with morbidity, renal osteodystrophy, and mortality. A number of reports have delineated an increased risk of all-cause and cardiovascular mortality in patients with disorders of mineral metabolism. Although not found in all studies, the association with decreased survival primarily involves increased phosphate, calcium, calcium x phosphate product, and/or parathyroid hormone levels. These in turn are associated with accelerated atherosclerosis, arterial calcification, and an increased risk of adverse cardiovascular outcomes and death [Block, 1998 (5); London, 2003 (6)].
Serum phosphorus exceeding 5.5 mg/dl and calcium phosphate product over 52 mg2/dl2 each correlate with an increased risk of mortality in dialysis patients [Block, 1998 (5)].
These findings have led to recent KDOQI (Kidney Disease Outcomes Quality Initiative) recommendations for a more vigorous control of serum phosphorus to between 2.5 and 5.5 mg/dl, while maintaining calcium phosphate product at less than 55 mg2/dl2 [K/DOQI clinical practice guidelines, 2003 (7)].
Because of growing concerns relating to the relationship among cardiovascular disease, vascular calcification, and abnormalities in bone and mineral metabolism, a careful process of evidence review and expert deliberation resulted in the 2003 K/DOQI guidelines on bone metabolism [K/DOQI clinical practice guidelines, 2003(7)].

Based upon this perspective, the following is an overview of some of the general recommendations for patients undergoing maintenance dialysis [K/DOQI clinical practice guidelines, 2003 (7); KDIGO guidelines 2009 (8)]
- Therapy of elevated phosphate levels (greater than 5.5 mg/dL [>1.8 mmol/L]) that is refractory to dialysis and diet can be initiated with either calcium or non-metal salt based phosphate binders.
- The use of a cocktail of oral phosphate binders is encouraged, with a limit of 1.5 grams of calcium salts (making a maximum total of 2 grams of elemental calcium per day in conjunction with dietary calcium intake).
- Calcium salts should be avoided in patients with sustained intact PTH levels of <150 pg/mL, or plasma calcium levels of >9.5 mg/dL (>2.37 mmol/L). Vitamin D compounds should also be avoided or terminated in patients with calcium levels greater 9.5 mg/dL (>2.37 mmol/L).
- Non-calcium-based phosphate binders are preferred in patients with severe vascular or soft-tissue calcifications.
- Plasma calcium levels should be maintained at the lower end of the normal range (8.4 to 9.5 mg/dL [2.1 to 2.35 mmol/L]).
- The calcium-phosphate product should be kept less than 55 mg2/mL2 (<4.4 mmol2/L2) by first focusing on controlling plasma phosphate.

The following table 1 summarizes some of the recommendations according to KDIGO guidelines 2009 (8).

**Table 1: Recommendations according to the KDIGO guidelines 2009. GFR = estimated glomerular filtration rate, a parameter for stratifying kidney function (KDIGO, 2009 (8)).**

| CKD stage | 3 | 4 | 5 |
|---|---|---|---|
| GFR (ml/min per 1.73 m²) | 30 - 59 | 15 - 29 | <15 |
| Serum Phosphate, target | normal range (0,81 - 1,45 mmol/l) | | lowering towards the normal range (≤ 1,45 mmol/l) |
| Lab test | 6-12 month | 3-6 month | monthly |
| Corrected total Calcium | normal range (2,20 - 2,65 mmol/l) | | |
| Lab test | 6-12 month | 3-6 month | monthly |
| iPTH | normal range | | 2 - 9 times the upper normal limit |
| Lab test | 6-12 months | 6-12 months | 3- 6 months |

The main consequences of hyperphosphatemia are cardiovascular complications, which are the main cause of death in patients suffering from chronic kidney failure. At local level these complications are manifested by alterations of the endothelium, accumulation of lipids, formation of clots and occlusion of the lumen.

Adherence to these guidelines mandates the use of a variety of different phosphate lowering agents in many patients if the central phosphate control targets are to be achieved. Approaches to the treatment of hyperphosphatemia by administering products with phosphate lowering activity (phosphate lowering agents) are available:
- calcium based binders, i.e. calcium acetate, calcium carbonate, calcium-magnesium-salts,
- aluminum based binders, i.e. aluminum chloride and aluminum hydrochloride,
- lanthanum carbonate
- iron containing phosphate binders (iron citrate, sucroferric oxyhydroxide)
all of them acting by *physico-chemical precipitation* of agent and phosphate taken in by diet and precipitating in the gastro-intestinal tract (i.e. classified as phosphate binders).
Moreover,
- sevelamer carbonate or sevelamer HCl (polymer)
are phosphate lowering agents which act by *physico-chemical absorption* of phosphate taken in by diet and being absorbed by the polymer during the gastro-intestinal passage.
The terms "phosphate lowering agents" and "phosphate binders" are used herein interchangeably.
Due to the mode of action, pill intake with meals is essential, high dosages are required and patient compliance is a pre-condition, but due to high tablet burden (3 to 6 tablets or capsules per meal) frequently insufficient. In consequence, up to 70% of CKD patients are still in hyperphosphatemia despite treatment with above mentioned phosphate lowering agents [Navaneethan, 2009 (9)] and do not meet above mentioned phosphate levels recommended by KDIGO and KDOQI [K/DOQI clinical practice guidelines, 2003 (7); KDIGO guideline, 2009 (8)]. Nicotinamide acts in a pharmacological, pharmaco-physiological mode of action by down-regulating NaPi2b cotransporters predominantly expressed in the small intestine. Extracellular phosphate homeostasis is achieved by the regulation of intestinal phosphate absorption as well as by regulation of phosphate excretion via the kidneys. Further, phosphate homeostasis is regulated by an integrated endogenous crosstalk involving kidney, bone and intestine (Ketteler, 2011 (58)). Extracellular phosphate homeostasis is achieved by the regulation of intestinal phosphate absorption as well as by regulation of phosphate excretion via the kidneys. Current knowledge suggests three different sodium-dependent phosphate cotransporters (NaPi2a, NaPi2c and NaPi2b) as well as two type 3 cotransporters (PiT1 and PiT2) being responsible for regulation of intestinal and renal phosphate regulation (Marks, 2010 (10), Suyama, 2012 (11)). NaPi2b cotransporters are essential for the active up-take of phosphate which contributes to ca. 50% of phosphate uptake into serum (Katai, 1999 (12)). The kidneys express four different phosphate cotransporters. Three of them (NaPi2a, NaPi2c, PiT2) are located in the proximal part of the tubule apparatus at the apical side of kidney epithelial cells (Forster, 2013 (56)). Their physiological role is the reabsorption of filtrated phosphate from the primary urine. Recently, the phosphate cotransporter NaPi2b was also detected in the kidney of rats (Suyama, 2012 (11)). In contrast to the cotransporters mentioned above, NaPi2b is expressed at the basolateral side of epithelial cells surrounding the urinary duct and it was suggested that the physiological role is to enhance basal phosphate excretion levels in the kidney. In line with this assumption, renal NaPi2b expression is strongly enhanced under high phosphorus diet (Suyama, 2012 (11)). Moreover, in a mouse model of adenine induced CKD, renal expression of NaPi2b was also significantly enhanced, while expression of NaPi2a and NaPi2c was reduced (Pulskens, 2015 (57)). A brief summary of the transport mechanisms is found in the following Table 2.

**Table 2: Active phosphate cotransporters in kidney and intestine. According to (Giral, 2009 (13); Marks, 2010 (10); Sabbagh, 2011 (14), Suyama, 2012 (11))**

| | Distribution | % of PO4 flow rate | Physiological regulators | Pharmacological regulators |
|---|---|---|---|---|
| NaPi2a | Proximal renal tubule BBM (S1-S3) | ≤ 70% (of renal reabsorption) | PTH (↓) FGF23 (↓), High PO₄ (↓) | PFA (↓) |
| NaPi2b | BBM of the small intestine | ≤ 50% (of intestinal absorption) | Calcitriol (↑), High PO₄ (↓), Low PO₄ (↑), FGF23 (indirect ↓) | Nicotinamide (↓) PFA (↓) |
| | Epithelial cells of the urinary duct | Proportion of renal excretion is not defined yet | High PO₄ (↑) | |
| NaPi2c | Proximal renal tubule BBM (S1) | ≥ 30% (of renal reabsorption) | FGF23 (↓), High dietary PO₄ (↓), High dietary Mg²⁺ (↑) | PFA (↓) |
| NaPi3 PiT1 | Duodenal and jejunal BBM | No data | FGF23 (↓), High dietary PO₄ (↓), Metabolic acidosis (↑) | No data |
| NaPi3 PiT2 | Proximal renal tubule BBM | 3 - 40% | FGF23 (↓), high dietary PO₄ (↓), Metabolic acidosis (↑) | No data |

| | | | | |
|---|---|---|---|---|
| BBM = Brush Border Membrane, MEPE = matrix extracellular phosphoglycoprotein, , FGF23 = Fibroblast growth factor 23, Mg²⁺ = Magnesium, NaPi = Sodium phosphate cotransporter, PFA = Phosphonoformic acid, PiT = Sodium dependent phosphate cotransporter, PO₄ = Phosphate, S = Segment, VDR = Vitamin D receptor, PTH = Parathormone | | | | |

It has been shown that nicotinamide can be effective in lowering elevated phosphate levels in animals with experimentally induced CKD (Eto, 2005 (18)) and in humans with end stage renal disease on dialysis (Takahashi, 2004 (19), Medice, 2015 (36)).
Inhibition of renal NaPi2a and NaPi2c protein expression either in double knockout mice (Marks, 2010 (10)) or via FGF23 (Gattineni, 2009 (15)) induces severe hypophosphatemia by blockade of tubular phosphate reabsorption in the kidneys.
Sodium dependent phosphate cotransporter NaPi2b was shown to be responsible for around 50% of gastrointestinal phosphate absorption (Katai, 1999 (12)). Beneath this transcellular transport mechanism passive phosphate diffusion is also important in intestinal phosphate uptake.
The expression of intestinal NaPi2b is blocked by a phosphate-rich diet (Hattenhauer, 1999 (16)). A low-phosphate diet (Giral, 2009 (13); Hattenhauer, 1999 (16)) or an increase in serum calcitriol (Xu, 2002 (17)) increases the expression of the cotransporter. FGF23 was shown to exert an indirect inhibitory action on intestinal NaPi2b expression via inhibition of renal 1α-hydroxylase activity and therefore decreasing Calcitriol levels (Marks, 2010 (10)).
A decrease in the absorption of phosphate from the small intestine, due to inhibition of the phosphate cotransporter NaPi2b, can be regarded as a new mechanism of action in the reduction of phosphate concentrations. Intraperitoneal administration of nicotinamide blocks the expression of NaPi2b (Eto, 2005 (18)) and inhibits the gastrointestinal absorption of phosphate (Katai, 1999 (12)). It has not been established whether the functional cotransporter is also directly inhibited.
It has been shown that nicotinamide can be effective in lowering elevated phosphate levels in animals (Eto, 2005 (18)) and in humans, and an overview is given in Table 3.

**Table 3: Overview of nicotinamide studies in CKD patients**

| Source | n | Patients | average dose (mg/d) | dose range (mg/d) | treatment duration (weeks) |
|---|---|---|---|---|---|
| Takahashi et al. 2004 (19) | 65 | Hemodialysis | 1080 | 500 - 1750 | 12 |
| Rahmouni et al. 2005 (20) | 10 | Hemodialysis | 720 | 500 - 1000 | 9 |
| Cheng et al. 2008 (21) | 33 | Hemodialysis | 1500 | 500 - 1500 | 8 |
| Young et al. 2009 (22) | 8 | Peritonealdialysis | 1000 | 500 - 1500 | 8 |

However, the bioavailability and clinical efficacy and safety of modified release nicotinamide (MR-NA) has never been studied and systematically evaluated.

Further, phosphate homeostasis is regulated by an integrated endogenous crosstalk involving kidney, bone and intestine (Ketteler, 2011 (58)). Decline of kidney function results in a cascade of pathophysiological events that result in mineral and bone disorder (MBD). MBD is characterized by progressive development of secondary hyperparathyroidism, arterial calcification, altered arterial function and abnormal bone metabolism. These changes contribute to further loss of kidney function, bone demineralization, fractures and high cardiovascular morbidity and mortality (KDIGO, 2009 (8)).
Subtle phosphate retention due to loss of filtering nephrons in early chronic kidney disease (CKD) plays a central role in the development of CKD-MBD (Block, 2013 (61)). Retention of phosphate signals the phosphaturic hormones parathyroid hormone (PTH) and fibroblast growth factor 23 (FGF23), both resulting in increased fractional phosphate excretion through the kidneys (Gutierrez, 2005 (62)). Additionally, phosphate retention inhibits renal synthesis of 1,25 dihydroxyvitamin D (1,25 (OH)2D), resulting in reduced intestinal absorption of phosphate (Marks, 2006 (63)). As a consequence, phosphaturic hormones and 1,25(OH)2D display characteristic changes in early kidney disease while blood phosphate levels remain in the normal range until CKD stage 3-4 followed by a strong exponential increase in advanced stages, especially in CKD stage 4/5.
CKD is associated with a strongly increased risk for cardiovascular disease (CVD) (Go, 2004 (64)) and thus, cardiovascular morbidity and mortality is strongly increased in CKD (Kestenbaum, 2005 (65)) and in patients with end stage renal disease (Block, 2004 (66)). In Germany, the 5 year survival rate of patients on hemodialysis is only 38% (Frei, 2008 (67)). This extremely high mortality is driven by a 30- to 100-fold increase in age-, gender-, and race-adjusted cardiovascular mortality rates (Foley, 1998 (68)). Altered mineral metabolism with raised blood phosphate levels (hyperphosphatemia) is the strongest independent predictor and risk factor for all cause and cardiovascular mortality in CKD patients (Kestenbaum, 2005 (65)). Beneath hyperphosphatemia, CKD patients exhibit other risk factors for cardiovascular disease. Dyslipidemia is a very common comorbidity of CKD patients. Typically CKD patients have high levels of triglycerides and especially patients with nephrotic syndrome exhibit a considerable increase of low-density lipoproteins (LDL) (Mikolasevic, 2017 (23)). LDL lowering was demonstrated to reduce cardiovascular mortality in CKD patients (Baigent, 2011(24)) as well as in diabetic patients on hemodialysis (März, 2011(25)). Dyslipidemia results in the classical picture of atherosclerosis, defined by the formation of lipid deposits forming fatty streaks in the lumen of blood vessels, growing up to plaques of variable size that result in occlusion of vessels (Amann, 2008 (26)).
In contrast, high serum phosphate directly results in dystrophic calcification of the medial smooth muscle layer of blood vessels. Additionally, high blood phosphate levels result in secondary calcification of intima plaques resulting from lipid deposition (Moe, 2004 (69)). Thus both, hyperphosphatemia as well as dyslipidemia synergistically affect severity of arteriosclerotic calcification of the intima of blood vessels. Moreover, severity as well as frequency of secondary atherosclerotic calcifications is more frequent in CKD patients compared to the age matched general population and can be regarded as special complication of a comorbid condition in CKD patients characterized by dyslipidemia as well as hyperphosphatemia (Amann, 2008 (26)).
Beneath alterations in LDL cholesterin, dyslipidemia in CKD patients is also characterized by an elevation of blood levels of lipoprotein(a) (LP(a)). This is an LDL-like lipoprotein which contains covalently bound apolipoprotein(a) (Apo(a)) that distinguishes it from LDL. Because of its strong homology to plasma protease zymogene plasminogen, Apo(a) competes with plasminogen for plasminogen receptors, fibrinogen, and fibrin. These effects result in promoted thrombogenesis due to fibrinolysis inhibition (Mikolasevic, 2017 (23)). CKD patients exhibit markedly elevated concentrations of Lp(a) (Haffner, 1992 (27)) as well as increased concentrations of Apo(a)
(Trenkwalder, 1997 (28)). In CKD patients, high serum levels of Lp(a) are inversely correlated with all-cause death and acute coronary syndrome, indicating Lp(a) as an independent risk factor for cardiovascular events (Konishi, 2016 (70)). Moreover, in an prospective cohort study it was shown, that patients with high levels of Lp(a) have a significantly raised risk for the development of CKD over a median follow-up period of 10 years (Yun, 2016 (71)). Thus, current evidence suggests that high levels of Lp(a) trigger both development and progression of CKD as well as elevated cardiovascular morbidity and mortality in patients with advanced CKD.
Lp(a) is a low density lipoprotein complexed with Apo(a). Apo(a) is produced almost exclusively in the liver and Lp(a) plasma levels highly correlate with Apo(a) production (Kostner, 2013 (72)). Up to date, pharmacological interventions to lower Lp(a) are very limited. Treatment with an PCSK9 inhibitor reduces Lp(a) by around 35% (Kotani, 2017 (29). In addition nicotinic acid was shown to reduce Lp(a) also up to 35% (Carlson, 1989 (30)).
Nicotinic acid reduces Lp(a) plasma levels probably due to inhibition of hepatic Apo(a) gene expression (Chennamsetty, 2012 (31)). In addition this pharmacological action is probably linked to binding of nicotinic acid to the G-protein-coupled receptor GPR109A (Digby, 2012 (32)). It is not known whether nicotinamide also has the potential to reduce Lp(a) plasma levels.

Therefore there is still a need for further development of improved methods of preventing and/or treating elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure.

### Description of invention

The invention addresses the problem of hyperphosphatemia and/or dyslipidemia resulting from chronic kidney failure (CKD). The invention provides a pharmaceutical preparation comprising a pharmaceutically effective amount of modified release nicotinamide for prophylaxis and/or treatment of hyperphosphatemia and/or dyslipidemia resulting particularly from chronic kidney failure (CKD) as well as for the treatment and prevention of End-Stage Renal Disease (ESRD). The pharmaceutical preparation is administered preferably via the oral route or the parenteral route. The invention further addresses the problem of limited efficacy of available treatment options in terms of reduction of blood phosphate levels in patients particularly with CKD 3-5, as dietary modifications of phosphate intake as well as treatment with phosphate binders are inefficient in the reduction of phosphate burden in moderate CKD (Sprague, 2009 (59), Oliveira, 2010 (60)). The invention also provides a pharmaceutical preparation comprising a pharmaceutically effective amount of modified release nicotinamide for prophylaxis and/or treatment of hyperphosphatemia resulting particularly from CKD stages 3-5.

The inventors particularly also found an efficient reduction of elevated serum phosphate levels in patients with chronic kidney disease due to a dual mode of action. The known pharmacological basis for the reduction of elevated serum phosphate levels is linked to the nicotinamide induced reduction of phosphate cotransporter NaPi2b in the intestine, resulting in reduced absorption of phosphate from food. The invention shows that nicotinamide additionally reduces renal expression of cotransporter NaPi2b in individuals with residual renal function, resulting in enhanced excretion of phosphate via the kidneys. This dual mode of action results in a stronger reduction of elevated phosphate levels compared to the treatment with conventional phosphate binders that act solely by binding of phosphate from ingested food in the intestine.

This invention involves the administration of a pharmaceutically effective quantity of nicotinamide in a modified release formulation.

In a first aspect the present invention relates to a pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, wherein about 25 - 55 % by weight of the nicotinamide is released from the pharmaceutical preparation at a pH of about 0.5 to about 1.5 at a time of about 2 hours.

In a second aspect the present invention relates to a pharmaceutical preparation comprising modified release nicotinamide, wherein about 25 - 55 % by weight of the nicotinamide is released from the pharmaceutical preparation at a pH of about 0.5 to about 1.5 at a time of about 2 hours.

Furthermore disclosed is a kit-of-parts, comprising the pharmaceutical preparation of the second aspect and at least one phosphate binder.

Further embodiments and advantages of the invention can be taken form the following description, figures, the examples as well as the dependent claims, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

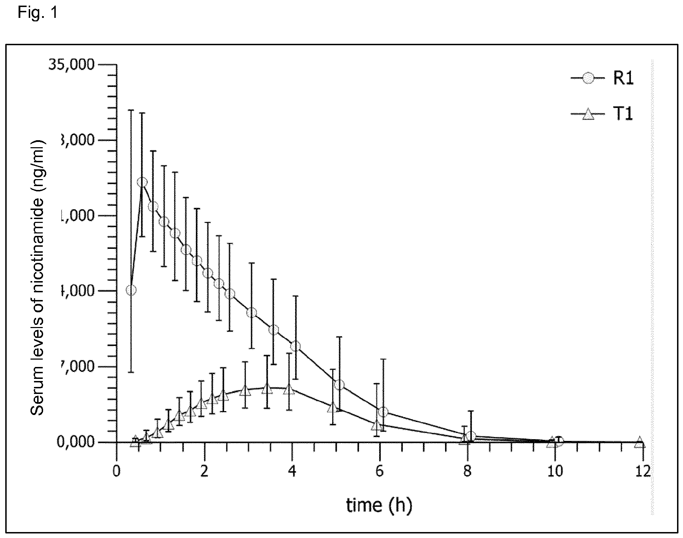

Fig. 1 shows a comparison between the bioavailability of immediate release nicotinamide and modified release nicotinamide in dosages of 1000mg in 24 healthy subjects undergoing a randomized, open-label, single dose, 2-treatment 4-period, cross-over study in fasting and fed state.

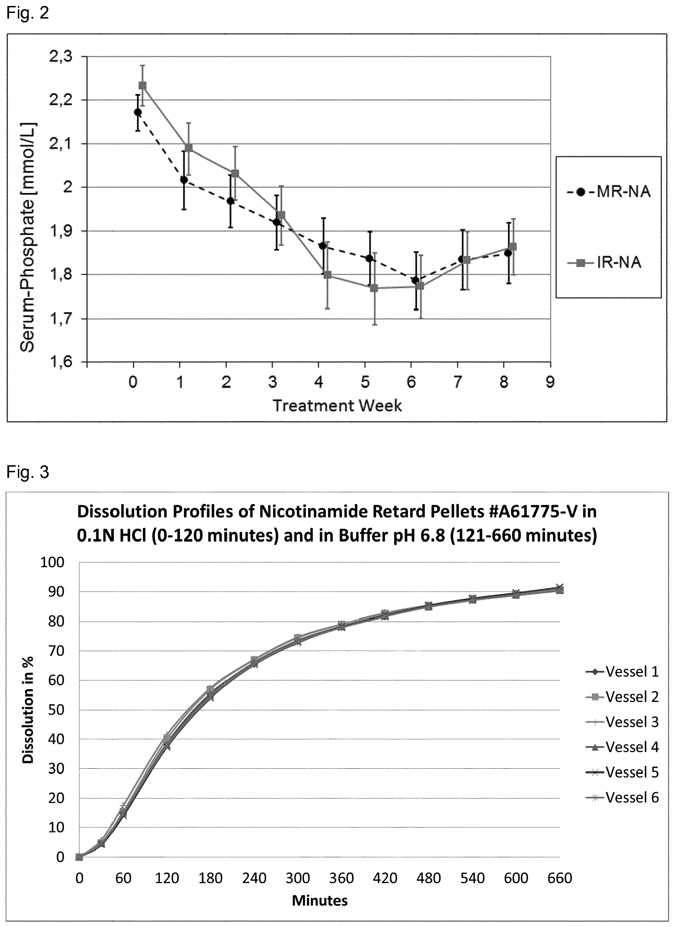

In Fig. 2 a comparison regarding serum phosphate levels between immediate release nicotinamide 1,000mg per day (IR-NA) given in three dosages per day (250mg-500mg-250mg p.o.) and modified release nicotinamide 1,000mg per day (0mg-1000mg-0mg p.o.; MR-NA) is shown.
Fig. 3 shows a comparison of release kinetics of six different preparations of nicotinamide retard pellets.

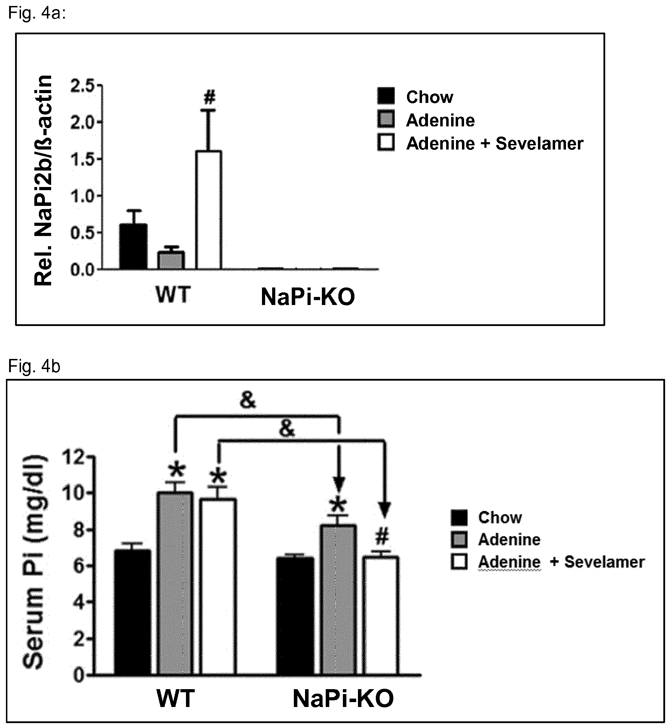

Fig. 4a illustrates quantification of NaPi2b protein expression in a mouse model of chronic kidney disease. In wild type mice (WT) adenine induced CKD resulted in small reductions of the NaPi2b phosphate cotransporter while treatment with the phosphate binder sevelamer resulted in a strong upregulation of NaPi2b protein expression.
Fig. 4b represents serum phosphate levels in two different strains of mice with experimentally induced CKD. In wild type mice (WT) adenine induced CKD resulted in a significant rise of phosphate levels. Treatment with the phosphate binder sevelamer did not lower elevated serum phosphate. In contrast sevelamer treatment in NaPi2b-Knock out mice (NaPi-KO) resulted in normalization of elevated phosphate levels, indicating that the lack of phosphate reduction in wild type animals depends to the enhanced expression of phosphate cotransporter NaPi2b.

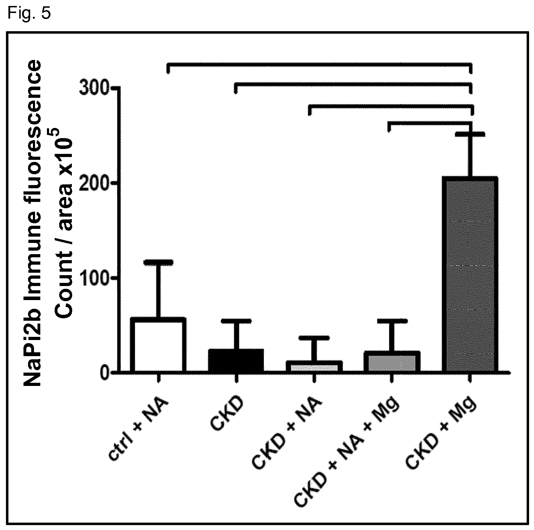

Fig. 5 shows results obtained in present Example 3. In a mouse model of surgically induced CKD, treatment with the phosphate binder magnesium carbonate (Mg) resulted in a strong enhancement of NaPi2b protein expression. This upregulation was completely abolished under combined treatment with nicotinamide (NA) and phosphate binder.

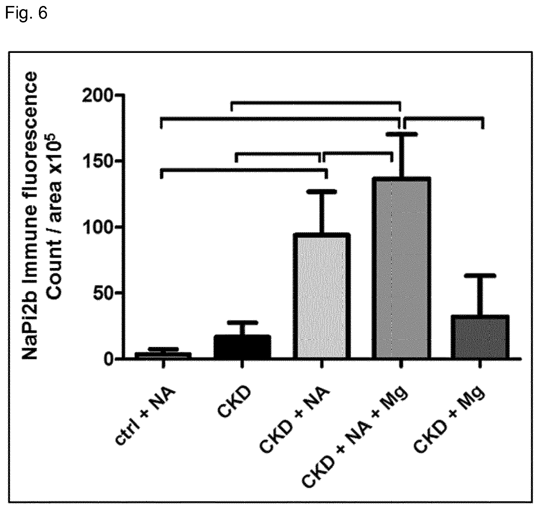

Fig. 6 depicts further results obtained in Example 3. Within the same mouse model of surgically induced CKD, treatment with nicotinamide resulted in a strong increase of NaPi2b protein expression in the kidneys. Combined treatment of nicotinamide and the phosphate binder magnesium carbonate further enhanced renal NaPi2b. Treatment with magnesium carbonate alone had no significant effects on renal NaPi2b-expression.

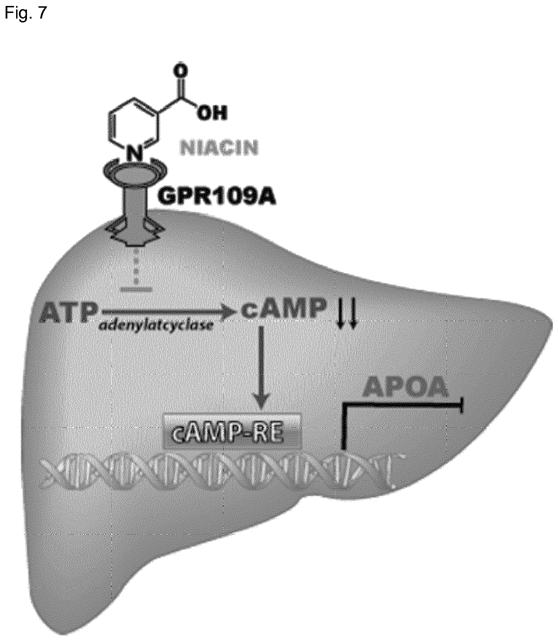

Fig. 7 shows a schematic of the supposed mode of action of nicotinic acid in reduction of Lp(a). Nicotinic acid binds specifically to the nicotinic acid receptor GRP109A (Tunaru 2005 (33)). After ligand binding the G-protein-coupled receptor inhibits intracelluar adenylatcylases that catalyze cyclic adenosine monophosphate generation (cAMP) from adenosine triphosphate (ATP). The translation of the apoprotein A gene is inhibited as the promotor region of the gene contains c-AMP response elements (cAMP-RE) (Gouni-Berthold, 2013 (34)).

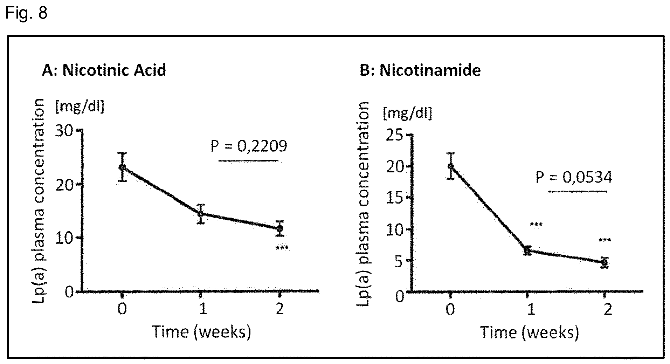

Fig. 8 refers to results obtained in present Example 5 and shows the reduction of Lp(a) plasma levels in transgenic Apo(a) mice treated either with 1% nicotinic acid (A) or nicotinamide (B). After 1 week of treatment only nicotinamide reduced Lp(a) levels significantly. After 2 weeks of treatment Lp(a) plasma levels were more than 50% lower compared to nicotinic acid and more than 200% lower compared to baseline.

### Definitions

Unless defined otherwise, technical and scientific terms used herein have the same meaning as commonly understood by one of ordinary skill in the art to which this invention belongs. The numerical figures provided herein like the unit doses of nicotinamide have to be understood as covering also "about" values.

The phosphate binders of the invention are also named phosphate lowering agents and are known in the art per se. According to the invention, also other phosphate binders acting in lowering the phosphate level can be used within the scope of the invention. The terms "phosphate lowering agents" and "phosphate binders" are used herein, within the scope of the invention, interchangeably.

A pharmaceutical preparation comprising modified release nicotinamide is a pharmaceutical preparation comprising nicotinamide in which the whole dose of the nicotinamide contained in the pharmaceutical preparation is not released directly upon taking of the pharmaceutical preparation, but is released upon and/or over a certain time, i.e. is in an extended release preparation/ a sustained release preparation. It shows a slower release of the nicotinamide than a conventional-release dosage form administered by the same route, i.e. an immediate release preparation.

A pharmaceutically effective amount of nicotinamide, e.g. modified release nicotinamide, can be an amount of nicotinamide in the pharmaceutical preparation that can achieve a therapeutic response or desired effect in some fraction of the subjects taking the pharmaceutical preparation.

With regard to the present invention, elevated phosphate levels are phosphate levels which exceed those recommended by medical guidelines, e.g. serum phosphate levels exceeding about 5.5 mg/dl and/or with serum phosphate levels about ≥ 1.78mmol/l.

In the present invention, dyslipidemia is represented by an abnormal amount of lipids (e.g. triglycerides, cholesterol, fat phospholipids) or substances derived thereof, e.g. lipoproteins, in the patient, particularly in the blood. According to certain embodiments, it refers to a dysregulation of lipid metabolism, particularly elevation of serum Lipoprotein(a) (Lp(a)) levels,

In a first aspect the present invention relates to a pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, wherein about 25 - 55 % by weight, preferably about 27 to about 45 % by weight, of the nicotinamide is released from the pharmaceutical preparation at a pH of about 0.5 to about 1.5, e.g. about 1.0 to about 1.2, e.g. about 1.0, at a time of about 2 hours. According to certain embodiments, at least about 60 % by weight, preferably at least about 65 % by weight, further preferably at least about 70 % by weight, of the nicotinamide is release from the pharmaceutical preparation after dissolution at a pH of about 0.5 to about 1.5, e.g. about 1.0 to about 1.2, for a time of about 2 hours and subsequent dissolution at a pH of about 6.5 to about 7.5, e.g. about 6.7 to about 7.0, e.g. about 6.8, for about 4 hours. Thus, the total treatment time at a pH of about 0.5 to about 1.5, e.g. about 1.0 to about 1.2, e.g. about 1.0, and subsequently at a pH of about 6.5 to about 7.5, e.g. about 6.7 to about 7.0, e.g. about 6.8, for the release of at least about 60 % by weight, preferably at least about 70 % by weight, of the nicotinamide is about 6 hours. Particularly, the nicotinamide is release at a pH of about 0.5 to about 1.5, and optionally subsequent further at a pH of about 6.5 to about 7.5 *in vitro,* further particularly at normal pressure (101325 Pa) and a room temperature of about 20 to about 25°C, e.g. about 22 to about 23°C, e.g. about 22°C. According to certain embodiments, about 15 to about 40 % by weight, preferably 17.5 to 37.5 % by weight of the nicotinamide is released from the pharmaceutical preparation at a time of about 1.5 hours, and/or about 40 to about 70 % by weight, preferably about 42.5 to about 67.5 % by weight, further preferably about 45 to about 65 % by weight of the nicotinamide is released from the pharmaceutical preparation at a time of about 3 hours, and/or about 65 to about 95 % by weight, preferably about 67.5 to about 92.5 %by weight, further preferably about 70 to about 90 % by weight of the nicotinamide is released from the pharmaceutical preparation at a time of about 7 hours after dissolution at a pH of about 0.5 to about 1.5, e.g. about 1.0 to about 1.2, e.g. about 1.0, for a time of about 2 hours and subsequent dissolution at a pH of about 6.5 to about 7.5, e.g. about 6.7 to about 7.0, e.g. about 6.8. Thus, according to certain embodiments, about 15 to about 40 % by weight, preferably 17.5 to 37.5 % by weight of the nicotinamide is released from the pharmaceutical preparation at a time of about 1.5 hours at a pH of about 0.5 to about 1.5, e.g. about 1.0 to about 1.2, e.g. about 1.0. According to certain embodiments, the release of the nicotinamide is measured in vitro by online monitoring. According to certain embodiments, the pharmaceutical preparation is put into a first container containing 0.1 N HCl at a pH of about 1.0 for about 2 hours, taken out after about 2 hours and immediately afterwards placed into a second container containing a 0.05 N potassium dihydrogen phosphate buffer at a pH of about 6.8 for measuring the release of nicotinamide. According to certain embodiments, the first container only contains 0.1 N HCl and/or the second container contains only 0.05 N potassium dihydrogen phosphate buffer. Instead of 0.1 N HCl and/or 0.05 N potassium dihydrogen phosphate buffer also other suitable acids and/or buffers can be used for providing a pH of about 0.5 to about 1.5, e.g. about 1.0 to about 1.2, e.g. about 1.0, and/or a pH of about 6.5 to about 7.5, e.g. about 6.7 to about 7.0, e.g. about 6.8. According to certain embodiments, the nicotinamide is released in an in vitro test wherein the tablet is stirred or not stirred in the respective container. According to certain embodiments, the release of nicotinamide is measured in vitro with a basket test at 100 rpm in line with European Pharmacopoeia, Ph. Eur. 2.9.3.

According to certain embodiments, the pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, is used in a method of preventing and/or treating of elevated serum phosphate levels (hyperphosphatemia) and dyslipidemia. According to certain embodiments, the pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, is used in a method of preventing and/or treating of elevated serum phosphate levels (hyperphosphatemia). According to certain embodiments, the pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, is used in a method of preventing and/or treating of dyslipidemia.

The invention is also directed in a fourth aspect to a method of preventing and/or treating elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, using a pharmaceutical preparation comprising a pharmaceutically effective amount of modified release nicotinamide, wherein about 25 - 55 % by weight, preferably about 27 to about 45 % by weight, of the nicotinamide is released from the pharmaceutical preparation at a pH of about 0.5 to about 1.5, e.g. about 1.0 to about 1.2, e.g. about 1.0,at a time of about 2 hours. Embodiments of this method correspond to embodiments of the first aspect of the present invention. Particularly, the nicotinamide is release at a pH of about 0.5 to about 1.5, and optionally subsequent further at a pH of about 6.5 to about 7.5 *in vitro,* further particularly at normal pressure and a room temperature of about 20 to about 25°C, e.g. about 22 to about 23°C, e.g. about 22°C. According to certain embodiments, about 15 to about 40 % by weight, preferably 17.5 to 37.5 % by weight of the nicotinamide is released from the pharmaceutical preparation at a time of about 1.5 hours, and/or about 40 to about 70 % by weight, preferably about 42.5 to about 67.5 % by weight, further preferably about 45 to about 65 % by weight of the nicotinamide is released from the pharmaceutical preparation at a time of about 3 hours, and/or about 65 to about 95 % by weight, preferably about 67.5 to about 92.5 %by weight, further preferably about 70 to about 90 % by weight of the nicotinamide is released from the pharmaceutical preparation at a time of about 7 hours after dissolution at a pH of about 0.5 to about 1.5, e.g. about 1.0 to about 1.2, e.g. about 1.0, for a time of about 2 hours and subsequent dissolution at a pH of about 6.5 to about 7.5, e.g. about 6.7 to about 7.0, e.g. about 6.8. Thus, according to certain embodiments, about 15 to about 40 % by weight, preferably 17.5 to 37.5 % by weight of the nicotinamide is released from the pharmaceutical preparation at a time of about 1.5 hours at a pH of about 0.5 to about 1.5, e.g. about 1.0 to about 1.2, e.g. about 1.0. According to certain embodiments, the release of the nicotinamide is measured in vitro by online monitoring. According to certain embodiments, the pharmaceutical preparation is put into a first container containing 0.1 N HCl at a pH of about 1.0 for about 2 hours, taken out after about 2 hours and immediately afterwards placed into a second container containing a 0.05 N potassium dihydrogen phosphate buffer at a pH of about 6.8 for measuring the release of nicotinamide. According to certain embodiments, the first container only contains 0.1 N HCl and/or the second container contains only 0.05 N potassium dihydrogen phosphate buffer. Instead of 0.1 N HCl and/or 0.05 N potassium dihydrogen phosphate buffer also other suitable acids and/or buffers can be used for providing a pH of about 0.5 to about 1.5, e.g. about 1.0 to about 1.2, e.g. about 1.0, and/or a pH of about 6.5 to about 7.5, e.g. about 6.7 to about 7.0, e.g. about 6.8. According to certain embodiments, the nicotinamide is released in an in vitro test wherein the tablet is stirred or not stirred in the respective container. According to certain embodiments, the release of nicotinamide is measured in vitro with a basket test at 100 rpm in line with European Pharmacopoeia, Ph. Eur. 2.9.3.

According to certain embodiments, the method of preventing and/or treating elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, using a pharmaceutical preparation comprising a pharmaceutically effective amount of modified release nicotinamide, is a method of preventing and/or treating of elevated serum phosphate levels (hyperphosphatemia) and dyslipidemia. According to certain embodiments, the method is a method of preventing and/or treating of elevated serum phosphate levels (hyperphosphatemia). According to certain embodiments, the method is a method of preventing and/or treating of dyslipidemia.

Besides nicotinamide the pharmaceutical preparation comprising a pharmaceutically effective amount of modified release nicotinamide for use in a method of preventing and/or treating of elevated serum phosphate levels and/or dyslipidemia, particularly dysregulation of lipid metabolism, particularly elevation of serum Lipoprotein(a) (Lp(a)) levels, as well as the pharmaceutical preparation used in the fourth aspect, can comprise further constituents which are not particularly restricted, like e.g. at least one pharmaceutically acceptable carrier, at least one modified release agent, and/or other excipients like antiadherents, binders, coatings, colors, disintegrants, flavors, fillers, diluents, glidants, lubricants, preservatives, sorbents, sweeteners and/or vehicles which are not particularly restricted. According to certain embodiments, the pharmaceutical preparation comprising a pharmaceutically effective amount of modified release nicotinamide for use in a method of preventing and/or treating of elevated serum phosphate levels, as well as the one used in the corresponding method of the second aspect, comprises at least one modified release agent.

According to certain embodiments, the pharmaceutical preparation for use in a method of preventing and/or treating elevated serum phosphate levels and/or dyslipidemia, particularly dysregulation of lipid metabolism, particularly elevation of serum Lipoprotein(a) (Lp(a)) levels, as well as the pharmaceutical preparation used in the fourth aspect, comprises a formulation comprising nicotinamide covered with a modified release coating. According to certain embodiments, the formulation comprising nicotinamide is in the form of pellets, i.e. comprises one or more pellets, e.g. a multitude of pellets. The pellets can then be comprised in a suitable dosage form, e.g. a capsule. The modified release coating is not particularly restricted and can be suitably set by the skilled person based on the release values of nicotinamide.

According to certain embodiments, the modified release coating comprises at least one binder and at least one modified release agent, preferably wherein the modified release agent comprises ethyl cellulose and/or hydroxypropyl methylcellulose, preferably ethyl cellulose and hydroxypropyl methylcellulose. According to certain embodiments, the modified release coating comprises ethyl cellulose and hydroxypropyl methylcellulose in a weight ratio of about 10: to about 20:1, preferably about 12:1 to about 16:1, e.g. about 14:1.

According to certain embodiments, the pharmaceutical preparation is in the form of tablets, capsules, oral preparations, powders, granules, lozenges, reconstitutable powders, syrups, solutions or suspensions.

According to certain embodiments, the pharmaceutical preparation is in the form of a capsule comprising pellets of modified release nicotinamide. The material of the capsule is not particularly restricted. According to certain embodiments the material of the capsule does not lead to an extended release of the pellets of modified release nicotinamide and preferably dissolved immediately at a pH of about 3 or less, e.g. about 2 or less or about 1.5 or less. According to certain embodiments, the capsule dissolves independently of the pH.

According to certain embodiments, the hyperphosphatemia and/or dyslipidemia, particularly dysregulation of lipid metabolism, particularly elevation of serum Lipoprotein(a) (Lp(a)) levels, result from chronic kidney failure, from of end-stage renal disease, and/or from hemodialysis.

According to certain embodiments, the subject of the present pharmaceutical preparation comprising a pharmaceutically effective amount of modified release nicotinamide for use in a method of preventing and/or treating elevated serum phosphate levels and/or dyslipidemia, particularly dysregulation of lipid metabolism, particularly elevation of serum Lipoprotein(a) (Lp(a)) levels, as well as a corresponding method of preventing and/or treating elevated serum phosphate levels and/or dyslipidemia, particularly dysregulation of lipid metabolism, particularly elevation of serum Lipoprotein(a) (Lp(a)) levels, is a mammal, particularly a human.

According to certain embodiments, the pharmaceutical preparation is administered parenterally or orally, preferably orally.

According to certain embodiments, the nicotinamide is to be administered in unit doses up to about 2000 mg per day, preferably in unit doses ranging from about 250 to about 2000 mg per day, further preferably from about 400 to about 1700 mg per day, even further preferably from about 500 to about 1500 mg per day.

According to certain embodiments, the nicotinamide is to be administered before, with and/or after meals, e.g. within 1 hour or within 30 minutes after meals, and/or before going to bed, e.g. within 1 hour or within 30 minutes before going to bed, independently from food intake and before and/or after hemodialysis or peritoneal dialysis treatment. According to certain embodiments, the pharmaceutical preparation comprising a pharmaceutically effective amount of modified release nicotinamide is administered once or twice daily independently from food intake, preferably once daily, further preferably before going to bed. Particularly with an administration once before going to bed a simultaneous taking of a phosphate binder can be avoided which might otherwise negatively affect the taking of the nicotinamide as an add-on.

According to certain embodiments, further at least one phosphate binder is administered. In this regard the at least one phosphate binder is not particularly limited.

The phosphate binder is not particularly restricted in this regard and those usually applied for the treatment of hyperphosphatemia and/or dyslipidemia, particularly dysregulation of lipid metabolism, particularly elevation of serum Lipoprotein(a) (Lp(a)) levels, can be applied. According to certain embodiments the phosphate binder is at least one selected from the group comprising
- calcium based binders, e.g. calcium acetate, calcium carbonate, calcium-magnesium-salts,
- aluminum based binders, e.g. aluminum chloride and aluminum-hydrochloride,
- lanthanum carbonate,
- iron containing phosphate binders, e.g. iron citrate, sucroferric oxyhydroxide, and / or
- sevelamer carbonate or sevelamer HCl (polymers).

Usual unit doses may vary according to phosphate binder applied, while, at least for some patients, the recommended daily dose (KDIGO 2009, DIMDI and WHO ATC defined daily doses) can be as follows,
- calcium based binders, e.g. calcium acetate (about 5600 - 6300 mg/d, e.g. ca. 6000 mg/d), calcium carbonate (ca. 4000 mg/d), calcium-magnesium-salts (about 4000 - 4500 mg/d, e.g. ca- 4226 mg/d), not exceeding the recommended daily unit dose of ca. 1500 mg elementary calcium per day
- aluminum-based binders, e.g. aluminum chloride, Al₉Cl₈(OH)₁₉ (about 900 - 1800 mg/d) and aluminum hydrochloride (about 1800 - 12000 mg/d), daily dose is e.g. ca. 1800 mg/d
- lanthanum carbonate, daily dose is e.g. about 3708 mg/d, and/or average daily dose is e.g. about 2250 mg/d
- iron containing phosphate binders, e.g. iron citrate, sucroferric oxyhydroxide, daily dose is ca. 7200 - 7500 mg/d
- sevelamer carbonate or sevelamer HCl (polymers), daily dose is ca. 5600 - 6400 mg/d.

The above recited doses may vary as written above and can be adjusted by the skilled person with respect to the disease and the individual patient to be treated as well as in relationship to the amount of the nicotinamide used and the kind of phosphate binder selected.

Regarding the dosage of the at least one phosphate binder and/or nicotinamide in a dosage form, reference can also be made to the established principles of pharmacology in human and veterinary medicine. Regarding the formulation of a ready-to-use medicament, reference can made to "Remington, The Science and Practice of Pharmacy", 22nd edition, 2013, pp. 777 - 1070. The contents thereof are incorporated by reference.

According to certain embodiments the pharmaceutical preparation comprising a pharmaceutically effective amount of modified release nicotinamide is administered at a time different from the administration of the at least one phosphate binder, preferably with a time difference of at least one hour, further preferably at least two hours, even further preferably at least three hours. It was found that phosphate binders like sevelamer can negatively affect the intestinal absorption of nicotinamide, presumably by complexing it. Thus, it is preferable that the phosphate binder and the pharmaceutical preparation comprising a pharmaceutically effective amount of modified release nicotinamide are given at different times. Preferably the time difference to the next taking of phosphate binder after the taking of the pharmaceutical preparation comprising a pharmaceutically effective amount of modified release nicotinamide is at least two hours, preferably at least three hours, particularly preferably at least four hours, so that the nicotinamide can be released over an extended time without an interference of phosphate binder. Preferably the pharmaceutical preparation comprising a pharmaceutically effective amount of modified release nicotinamide is taken before sleeping so that the time difference to the next taking of phosphate binder, which is usually taken together with a meal, is maximized. However, also other times of taking the pharmaceutical preparation comprising a pharmaceutically effective amount of modified release nicotinamide with sufficient difference to the time of taking phosphate binder is suitable. It is also not excluded that the pharmaceutical preparation comprising a pharmaceutically effective amount of modified release nicotinamide is taken together with the phosphate binder if the phosphate binder does essentially not negatively affect the nicotinamide uptake.

According to certain embodiments, the at least one phosphate binder is not sevelamer and/or a derivative thereof, e.g. sevelamer hydrochloride and/or sevelamer carbonate, particularly when administered concomitantly with the pharmaceutical preparation comprising a pharmaceutically effective amount of modified release nicotinamide. According to certain embodiments, the at least one phosphate binder is calcium acetate, calcium carbonate and/or lanthanum carbonate and/or an aluminum containing phosphate binder and/or a phosphate binder containing iron, as e.g. given above.

In a second aspect the present invention relates to a pharmaceutical preparation comprising modified release nicotinamide, particularly a pharmaceutically effective amount of modified release nicotinamide, wherein about 25 - 55 % by weight of the nicotinamide is released from the pharmaceutical preparation at a pH of about 0.5 to about 1.5 at a time of about 2 hours. According to certain embodiments, at least about 60 % by weight, preferably at least about 70 % by weight, of the nicotinamide is release from the pharmaceutical preparation after dissolution at a pH of about 0.5 to about 1.5, e.g. about 1.0 to about 1.2, e.g. about 1.0, for a time of about 2 hours and subsequent dissolution at a pH of about 6.5 to about 7.5, e.g. about 6.7 to about 7.0, e.g. about 6.8, for about 4 hours. Thus, the total treatment time at a pH of about 0.5 to about 1.5, e.g. about 1.0 to about 1.2, e.g. about 1.0, and subsequently at a pH of about 6.5 to about 7.5, e.g. about 6.7 to about 7.0, e.g. about 6.8, for the release of at least about 60 % by weight, preferably at least about 70 % by weight, of the nicotinamide is about 6 hours. According to certain embodiments, about 15 to about 40 % by weight, preferably 17.5 to 37.5 % by weight of the nicotinamide is released from the pharmaceutical preparation at a time of about 1.5 hours, and/or about 40 to about 70 % by weight, preferably about 42.5 to about 67.5 % by weight, further preferably about 45 to about 65 % by weight of the nicotinamide is released from the pharmaceutical preparation at a time of about 3 hours, and/or about 65 to about 95 % by weight, preferably about 67.5 to about 92.5 %by weight, further preferably about 70 to about 90 % by weight of the nicotinamide is released from the pharmaceutical preparation at a time of about 7 hours after dissolution at a pH of about 0.5 to about 1.5, e.g. about 1.0 to about 1.2, e.g. about 1.0, for a time of about 2 hours and subsequent dissolution at a pH of about 6.5 to about 7.5, e.g. about 6.7 to about 7.0, e.g. about 6.8. Thus, according to certain embodiments, about 15 to about 40 % by weight, preferably 17.5 to 37.5 % by weight of the nicotinamide is released from the pharmaceutical preparation at a time of about 1.5 hours at a pH of about 0.5 to about 1.5, e.g. about 1.0 to about 1.2, e.g. about 1.0. According to certain embodiments, the release of the nicotinamide is measured in vitro by online monitoring. According to certain embodiments, the pharmaceutical preparation is put into a first container containing 0.1 N HCl at a pH of about 1.0 for about 2 hours, taken out after about 2 hours and immediately afterwards placed into a second container containing a 0.05 N potassium dihydrogen phosphate buffer at a pH of about 6.8 for measuring the release of nicotinamide. According to certain embodiments, the first container only contains 0.1 N HCl and/or the second container contains only 0.05 N potassium dihydrogen phosphate buffer. Instead of 0.1 N HCl and/or 0.05 N potassium dihydrogen phosphate buffer also other suitable acids and/or buffers can be used for providing a pH of about 0.5 to about 1.5, e.g. about 1.0 to about 1.2, e.g. about 1.0, and/or a pH of about 6.5 to about 7.5, e.g. about 6.7 to about 7.0, e.g. about 6.8. According to certain embodiments, the nicotinamide is released in an in vitro test wherein the tablet is stirred or not stirred in the respective container. According to certain embodiments, the release of nicotinamide is measured in vitro with a basket test at 100 rpm in line with European Pharmacopoeia, Ph. Eur. 2.9.3.

The pharmaceutical preparation of the second aspect can be used as a pharmaceutical preparation comprising a pharmaceutically effective amount of modified release nicotinamide for use in a method of preventing and/or treating of elevated serum phosphate levels and/or dyslipidemia, particularly dysregulation of lipid metabolism, particularly elevation of serum Lipoprotein(a) (Lp(a)) levels of the first aspect, or in the corresponding method.

According to certain embodiments, the pharmaceutical preparation is in the form of tablets, capsules, oral preparations, powders, granules, lozenges, reconstitutable powders, syrups, solutions or suspensions.

Further constituents of the pharmaceutical preparation like at least one pharmaceutically acceptable carrier and/or other excipients are not particularly restricted. The pharmaceutical preparation can comprise pharmaceutically acceptable excipients like antiadherents; binders; coatings; network forming excipients; colours; disintegrants; flavors; fillers; diluents; glidants like fumed silica, talc, magnesium stearate and/or magnesium carbonate; lubricants like talc, silica and/or fats; preservatives like antioxidants, e.g. vitamin A, vitamin E, vitamin C, etc., the amino acids cysteine and methionine, citric acids and salts thereof, e.g. sodium citrate, and/or synthetic preservatives; sorbents, like desiccants; sweeteners; water stabilizers; antifungals and/or vehicles which preferably do not interact with the nicotinamide and/or at least one phosphate binder.

These excipients are well-known to the skilled person, e.g. from Remington, The Science and Practice of Pharmacy, 22nd Edition, 2012, volume 1: "The Science of Pharmacy", pages 1049-1070, which is incorporated herein by reference in regard to pharmaceutical excipients.

According to certain embodiments, the pharmaceutical preparation comprises a formulation comprising nicotinamide covered with a modified release coating. According to certain embodiments, the formulation comprising nicotinamide is in the form of pellets.

The modified release coating is not particularly restricted and can be suitably set by the skilled person based on the release values of nicotinamide. Regarding the formulation of such a modified release coating reference can made e.g. to Remington, The Science and Practice of Pharmacy", 22nd edition, 2013, pp. 981, 982, 989 -998. The contents thereof are incorporated by reference.

According to certain embodiments, the modified release coating comprises at least one binder and at least one modified release agent, preferably wherein the modified release agent comprises (meth)acrylate copolymers like ammonium methacrylate copolymers and methyl methacrylate copolymers; acrylate derivatives like ethyl acrylates; and/or cellulose derivatives like ethyl cellulose and hydroxypropyl methylcellulose, e.g. ethyl cellulose and hydroxypropyl methylcellulose. According to certain embodiments, the modified release coating comprises ethyl cellulose and hydroxypropyl methylcellulose in a weight ratio of about 10:1 to about 20:1, preferably about 12:1 to about 16:1, e.g. about 14:1.

The pellets can comprise one or more binders, like saccharides and their derivatives, e.g. disaccharides like sucrose, lactose; polysaccharides and their derivatives like starches, cellulose or modified cellulose like microcrystalline cellulose and cellulose ethers such as hydroxypropyl cellulose; sugar alcohols like xylitol, sorbitol and maltitol; proteins like gelatin; or synthetic polymers like polyvinyl pyrrolidone or polyethylene glycol, etc., e.g. microcrystalline cellulose, besides nicotinamide. The pellets covered with a modified release coating can further contain softening agents like dibutyl sebacate, tributyl citrate, triethyl citrate, acetyl triethyl citrate, etc., e.g. dibutyl sebacate, and/or separating agents and/or flow aids like glycerolmonostearate, talc and/or colloidal anhydrous silica. According to certain embodiments, the pellets comprise microcrystalline cellulose, ethyl cellulose, dibutyl sebacate, hydroxypropyl methylcellulose, glycerol monostearate, talc, and colloidal anhydrous silica.

According to certain embodiments, the pharmaceutical preparation is in the form of a capsule comprising pellets of modified release nicotinamide. The material of the capsule is not particularly restricted. According to certain embodiments the material of the capsule does not lead to an extended release of the pellets of modified release nicotinamide and preferably dissolved immediately at a pH of about 3 or less, e.g. about 2 or less or about 1.5 or less. According to certain embodiments, the capsule dissolves independently of the pH.

The capsule can be a hard capsule or a soft capsule, e.g. a hard capsule, e.g. formed of a capsule cap and a capsule body, both of which are not particularly restricted and which can e.g. contain pharmaceutically acceptable excipients as listed above regarding the pharmaceutical preparation. For example, the capsule cap can contain materials like gelatin; colors, e.g. titanium dioxide, indigo carmine, black iron oxide, and/or erythrosine; sodium lauryl sulfate; and/or purified water, and/or the capsule body can contain materials like gelatin; titanium dioxide; sodium lauryl sulfate; and/or purified water.

According to certain embodiments, the pharmaceutical preparation comprises from about 100 mg to about 1500 mg, preferably from about 100 mg to about 500 mg, e.g. about 250 mg of nicotinamide.

The present invention also relates in a third aspect to a kit-of-parts, comprising the pharmaceutical preparation of the second aspect and at least one phosphate binder. In such a kit-of-parts one dosage form can e.g. comprise the modified release nicotinamide and a further one can comprise at least one phosphate binder. The two or more dosage forms in the kit-of-parts can then each comprise at least one pharmaceutically acceptable carrier which can be the same or different.

The phosphate binder is not particularly restricted in this regard and those usually applied for the treatment of hyperphosphatemia and/or dyslipidemia, particularly dysregulation of lipid metabolism, particularly elevation of serum Lipoprotein(a) (Lp(a)) levels, can be applied. Also the phosphate binder can comprise at least one carrier and/or pharmaceutically acceptable excipients like antiadherents, binders, coatings, colours, disintegrants, flavors, fillers, diluents, glidants, lubricants, preservatives, sorbents, sweeteners, water stabilizers, antifungals and/or vehicles which preferably do not interact with the nicotinamide and/or at least one phosphate binder.

According to certain embodiments the phosphate binder is at least one selected from the group comprising
- calcium based binders, e.g. calcium acetate, calcium carbonate, calcium-magnesium-salts,
- aluminum based binders, e.g. aluminum chloride and aluminum-hydrochloride,
- lanthanum carbonate,
- iron containing phosphate binders, e.g. iron citrate, sucroferric oxyhydroxide, and / or
- sevelamer carbonate or sevelamer HCl (polymers).

Usual unit doses may vary according to phosphate binder applied, while, at least for some patients, the recommended daily dose (KDIGO 2009, DIMDI and WHO ATC defined daily doses) can be as follows,
- calcium based binders, e.g. calcium acetate (about 5600 - 6300 mg/d, e.g. ca. 6000 mg/d), calcium carbonate (ca. 4000 mg/d), calcium-magnesium-salts (about 4000 - 4500 mg/d, e.g. ca- 4226 mg/d), not exceeding the recommended daily unit dose of ca. 1500 mg elementary calcium per day
- aluminum-based binders, e.g. aluminum chloride, Al₉Cl₈(OH)₁₉ (about 900 - 1800 mg/d) and aluminum hydrochloride (about 1800 - 12000 mg/d), daily dose is e.g. ca. 1800 mg/d
- lanthanum carbonate, daily dose is e.g. about 3708 mg/d, and/or average daily dose is e.g. about 2250 mg/d
- iron containing phosphate binders, e.g. iron citrate, sucroferric oxyhydroxide, daily dose is ca. 7200 - 7500 mg/d
- sevelamer carbonate or sevelamer HCl (polymers), daily dose is ca. 5600 - 6400 mg/d.

The above recited doses may vary as written above and can be adjusted by the skilled person with respect to the disease and the individual patient to be treated as well as in relationship to the amount of the nicotinamide used and the kind of phosphate binder selected.

Regarding the dosage of the at least one phosphate binder and/or nicotinamide in a dosage form, reference can also be made to the established principles of pharmacology in human and veterinary medicine. Regarding the formulation of a ready-to-use medicament, reference can made to "Remington, The Science and Practice of Pharmacy", 22nd edition, 2013, pp. 777 - 1070. The contents thereof are incorporated by reference.

According to certain embodiments, the at least one phosphate binder in the kit-of-parts is not sevelamer and/or a derivative thereof, e.g. sevelamer hydrochloride and/or sevelamer carbonate, particularly when administered concomitantly with the pharmaceutical preparation comprising a pharmaceutically effective amount of modified release nicotinamide. According to certain embodiments, the at least one phosphate binder in the kit-of-parts is calcium acetate, calcium carbonate and/or lanthanum carbonate and/or an aluminum containing phosphate binder and/or a phosphate binder containing iron, as e.g. given above.

The kit-of-parts can be used for treating elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, particularly wherein said hyperphosphatemia and/or dyslipidemia, particularly dysregulation of lipid metabolism, particularly elevation of serum Lipoprotein(a) (Lp(a)) levels, result from chronic kidney failure, from of end-stage renal disease, and/or from hemodialysis. It can be administered parenterally and/or orally, e.g. one dosage form can be administered parenterally and one orally in case of a kit-of-parts.

Further disclosed is in a fifth aspect a pharmaceutical preparation comprising a pharmaceutically effective amount of modified release nicotinamide, e.g. as defined above with regard to the first aspect, for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, in patients in phases 4 and/or 5 of chronic kidney disease, excluding patients undergoing dialysis treatment, both particularly resulting from renal failure. According to certain embodiments, the patients have a glomerular filtration rate of 30 ml/min/1,73 m² or less and 10 ml/min/1,73 m² or more, preferably less than 30 ml/min/1,73 m² and more than 10 ml/min/1,73 m², and/or do not undergo dialysis treatment.

Also disclosed is in a sixth aspect a method of preventing and/or treating elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, using a pharmaceutical preparation comprising a pharmaceutically effective amount of modified release nicotinamide, e.g. as defined above with regard to the second aspect. According to certain embodiments, the methods is applied to patients having a glomerular filtration rate of 30 ml/min/1,73 m² or less and 10 ml/min/1,73 m² or more, preferably less than 30 ml/min/1,73 m² and more than 10 ml/min/1,73 m².

According to certain embodiments, the pharmaceutical preparation of the fifth aspect and/or in the sixth aspect is administered parenterally or orally, preferably orally.

Besides nicotinamide the pharmaceutical preparation comprising a pharmaceutically effective amount of nicotinamide for use in a method of the fifth aspect, as well as the pharmaceutical preparation used in the sixth aspect, can comprise further constituents which are not particularly restricted, like e.g. at least one pharmaceutically acceptable carrier and/or excipients like antiadherents; binders, like saccharides and their derivatives, e.g. disaccharides like sucrose, lactose; polysaccharides and their derivatives like starches, cellulose or modified cellulose like microcrystalline cellulose and cellulose ethers such as hydroxypropyl cellulose; sugar alcohols like xylitol, sorbitol and maltitol; proteins like gelatin; or synthetic polymers like polyvinyl pyrrolidone or polyethylene glycol, etc., e.g. microcrystalline cellulose; softening agents like dibutyl sebacate, tributyl citrate, triethyl citrate, acetyl triethyl citrate, etc., e.g. dibutyl sebacate, and/or separating agents and/or flow aids like glycerolmonostearate, talc and/or colloidal anhydrous silica; coatings; network forming excipients; colours; disintegrants; flavors; fillers; diluents; glidants like fumed silica, talc, magnesium stearate and/or magnesium carbonate; lubricants like talc, silica and/or fats; preservatives like antioxidants, e.g. vitamin A, vitamin E, vitamin C, etc., the amino acids cysteine and methionine, citric acids and salts thereof, e.g. sodium citrate, and/or synthetic preservatives; sorbents, like desiccants; sweeteners; water stabilizers; antifungals and/or vehicles which preferably do not interact with the nicotinamide and/or at least one phosphate binder.

These excipients are well-known to the skilled person, e.g. from Remington, The Science and Practice of Pharmacy, 22nd Edition, 2012, volume 1: "The Science of Pharmacy", pages 1049-1070, which is incorporated herein by reference in regard to pharmaceutical excipients.

According to certain embodiments, the pharmaceutical preparation of the fifth aspect and/or used in the sixth aspect is in the form of tablets, capsules, oral preparations, powders, granules, lozenges, reconstitutable powders, syrups, solutions or suspensions. According to certain embodiments, the pharmaceutical preparation comprises a formulation comprising nicotinamide which can be in the form of pellets, i.e. comprises one or more pellets, e.g. a multitude of pellets.

According to certain embodiments, the pharmaceutical preparation is in the form of a capsule comprising pellets of nicotinamide. The material of the capsule is not particularly restricted. According to certain embodiments the material of the capsule does not lead to an extended release of the pellets of nicotinamide and preferably dissolved immediately at a pH of about 3 or less, e.g. about 2 or less or about 1.5 or less. According to certain embodiments, the capsule dissolves independently of the pH.

The capsule can be a hard capsule or a soft capsule, e.g. a hard capsule, e.g. formed of a capsule cap and a capsule body, both of which are not particularly restricted and which can e.g. contain pharmaceutically acceptable excipients as listed above regarding the pharmaceutical preparation. For example, the capsule cap can contain materials like gelatin; colors, e.g. titanium dioxide, indigo carmine, black iron oxide, and/or erythrosine; sodium lauryl sulfate; and/or purified water, and/or the capsule body can contain materials like gelatin; titanium dioxide; sodium lauryl sulfate; and/or purified water.

According to certain embodiments of the pharmaceutical preparation of the fifth aspect and/or in the sixth aspect the subject is a mammal, particularly a human.

According to certain embodiments of the pharmaceutical preparation of the fifth aspect and/or in the sixth aspect, the nicotinamide is to be administered in unit doses up to about 2000 mg per day, preferably in unit doses ranging from about 250 to about 2000 mg per day, further preferably from about 400 to about 1700 mg per day, even further preferably from about 500 to about 1500 mg per day.

According to certain embodiments of the pharmaceutical preparation of the fifth aspect and/or in the sixth aspect, the nicotinamide is to be administered before, with and/or after meals, e.g. within 1 hour or within 30 minutes after meals, and/or before going to bed, e.g. within 1 hour or within 30 minutes before going to bed, independently from food intake and before and/or after hemodialysis or peritoneal dialysis treatment.

According to certain embodiments of the pharmaceutical preparation of the fifth aspect and/or in the sixth aspect, the pharmaceutical preparation comprising a pharmaceutically effective amount of nicotinamide is administered once or twice daily independently from food intake, preferably once daily, further preferably before going to bed. Particularly with an administration once before going to bed a simultaneous taking of a phosphate binder can be avoided which might otherwise negatively affect the taking of the nicotinamide as an add-on.

According to certain embodiments of the pharmaceutical preparation of the fifth aspect and/or in the sixth aspect, further at least one phosphate binder is administered.

The phosphate binder is not particularly restricted in this regard and those usually applied for the treatment of hyperphosphatemia and/or dyslipidemia, particularly dysregulation of lipid metabolism, particularly elevation of serum Lipoprotein(a) (Lp(a)) levels, can be applied. According to certain embodiments the phosphate binder is at least one selected from the group comprising
- calcium based binders, e.g. calcium acetate, calcium carbonate, calcium-magnesium-salts,
- aluminum based binders, e.g. aluminum chloride and aluminum-hydrochloride,
- lanthanum carbonate,
- iron containing phosphate binders, e.g. iron citrate, sucroferric oxyhydroxide, and / or
- sevelamer carbonate or sevelamer HCl (polymers).

Usual unit doses may vary according to phosphate binder applied, while, at least for some patients, the recommended daily dose (KDIGO 2009, DIMDI and WHO ATC defined daily doses) can be as follows,
- calcium based binders, e.g. calcium acetate (about 5600 - 6300 mg/d, e.g. ca. 6000 mg/d), calcium carbonate (ca. 4000 mg/d), calcium-magnesium-salts (about 4000 - 4500 mg/d, e.g. ca- 4226 mg/d), not exceeding the recommended daily unit dose of ca. 1500 mg elementary calcium per day
- aluminum-based binders, e.g. aluminum chloride, Al₉Cl₈(OH)₁₉ (about 900 - 1800 mg/d) and aluminum hydrochloride (about 1800 - 12000 mg/d), daily dose is e.g. ca. 1800 mg/d
- lanthanum carbonate, daily dose is e.g. about 3708 mg/d, and/or average daily dose is e.g. about 2250 mg/d
- iron containing phosphate binders, e.g. iron citrate, sucroferric oxyhydroxide, daily dose is ca. 7200 - 7500 mg/d
- sevelamer carbonate or sevelamer HCl (polymers), daily dose is ca. 5600 - 6400 mg/d.

The above recited doses may vary as written above and can be adjusted by the skilled person with respect to the disease and the individual patient to be treated as well as in relationship to the amount of the nicotinamide used and the kind of phosphate binder selected.

Regarding the dosage of the at least one phosphate binder and/or nicotinamide in a dosage form, reference can also be made to the established principles of pharmacology in human and veterinary medicine. Regarding the formulation of a ready-to-use medicament, reference can made to "Remington, The Science and Practice of Pharmacy", 22nd edition, 2013, pp. 777 - 1070. The contents thereof are incorporated by reference.

According to certain embodiments of the pharmaceutical preparation of the fifth aspect and/or in the sixth aspect, the pharmaceutical preparation comprising a pharmaceutically effective amount of nicotinamide is administered at a time different from the administration of the at least one phosphate binder, particularly if the at least one phosphate binder negatively affects the nicotinamide uptake, preferably with a time difference of at least one hour, further preferably at least two hours, even further preferably at least three hours. It was found that phosphate binders like sevelamer can negatively affect the intestinal absorption of nicotinamide, presumably by complexing it. Thus, according to certain embodiments the phosphate binder and the pharmaceutical preparation comprising a pharmaceutically effective amount of nicotinamide are given at different times, particularly if the at least one phosphate binder negatively affects the nicotinamide uptake. Preferably the time difference to the next taking of phosphate binder after the taking of the pharmaceutical preparation comprising a pharmaceutically effective amount of nicotinamide is at least one hour, preferably at least two hours, particularly preferably at least three hours, so that the nicotinamide can be released without an interference of phosphate binder. According to certain embodiments the pharmaceutical preparation comprising a pharmaceutically effective amount of nicotinamide is taken before sleeping so that the time difference to the next taking of phosphate binder, which is usually taken together with a meal, is maximized, e.g. also when nicotinamide is not immediately released from the pharmaceutical preparation. However, also other times of taking the pharmaceutical preparation comprising a pharmaceutically effective amount of nicotinamide with sufficient difference to the time of taking phosphate binder is suitable. It is also not excluded that the pharmaceutical preparation comprising a pharmaceutically effective amount of nicotinamide is taken together with the phosphate binder if the phosphate binder does essentially not negatively affect the nicotinamide uptake.

According to certain embodiments the at least one phosphate binder is not sevelamer and/or a derivative thereof, e.g. sevelamer hydrochloride and/or sevelamer carbonate, particularly when administered concomitantly with the pharmaceutical preparation comprising a pharmaceutically effective amount of nicotinamide. According to certain embodiments, the at least one phosphate binder is calcium acetate, calcium carbonate and/or lanthanum carbonate and/or an aluminum containing phosphate binder and/or a phosphate binder containing iron, as e.g. given above.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Example 1-1: Bioavailability of immediate release versus modified release nicotinamide

The invention is remarkable as one would expect treatment with immediate release nicotinamide (IR-NA) is sufficient for inhibition of the phosphate cotransporter NaPi2b. However, the inventors have shown that bioavailability, efficacy and safety profile of immediate release nicotinamide differs from modified release nicotinamide (MR-NA), resulting in differing pharmacokinetic profiles and clinical physiological effects in humans.

A modified release (MR) nicotinamide hard gelatin capsule containing modified release nicotinamide pellets was prepared using the following ingredients (with the approximate mass given per capsule):
- As active pharmaceutical ingredient, nicotinamide was used in a quantity of 250 mg. Further, the following excipients were used in the modified release pellets: microcrystalline cellulose (107.143 mg; binder), ethyl cellulose (17.857 mg; modified release agent), dibutyl sebacate (1.786 mg; softening agent), hydroxypropyl methylcellulose (1.276 mg; modified release agent), glycerol monostearate (0.918 mg; separating agent), micronized talc (0.204mg; separating agent), colloidal anhydrous silica (0.204 mg; separating agent, flow aid).
- The capsule consisted of a capsule cap and a capsule body. The capsule cap consisted of the following ingredients: gelatin (31.992 mg), titanium dioxide (0.384 mg), indigo carmine (0.200 mg), black iron oxide (0.157 mg), erythrosine (0.023 mg), sodium lauryl sulfate (0.077 mg), purified water (5.568 mg), giving a total mass of 38.4 mg for the capsule cap. The capsule body consisted of the following ingredients: gelatin (47.981 mg), titanium dioxide (1.152 mg), sodium lauryl sulfate (0.115 mg), purified water (8.352 mg), giving a total mass of 57.600 mg for the capsule body. The capsule was produced according to usual procedure for producing capsules.

For producing the pellets, the nicotinamide, microcrystalline cellulose and purified water (removed during drying) were weighed, mixed and granulated, extruded to form pellet rods, cut in wet form, spheronised, dried and classified. For the modified release coating the ethyl cellulose, dibutyl sebacate, hydroxypropyl methylcellulose and glycerol monostearate were mixed with ethanol (removed during drying) and suspended. The suspension was used for coating the pellets. After drying a 1:1 (w/w) mixture of the micronized talc and silicon dioxide (colloidal anhydrous silica) was dispersed onto the dried coated pellets. Afterwards the pellets were sieved and encapsulated. For example, for producing a batch containing 105.000 kg nicotinamide, 45.000 kg microcrystalline cellulose, 7.500 kg ethyl cellulose, 0.750 kg dibutyl sebacate, 0.536 kg hydroxypropyl methylcellulose, 0.386 kg glycerol monostearate and 0.170 kg of a 1:1 mixture of talc and colloidal anhydrous silica, 37.500 kg purified water and 60.000 kg ethanol are applied.

As comparative example, commercially available immediate release (IR) nicotinamide tablets (Nicotinsäureamid 5 x 200 mg JENAPHARM^{®} tablets) were used.

First, the MR pellets were tested for release of nicotinamide. Exemplary release profiles are shown in Fig. 3 for single charges. Shown are in vitro dissolution profiles of six different charges of modified release (MR) nicotinamide under pH conditions resembling the intestinal tract in six different dissolution preparations (vessels). The measurement was conducted in vitro using a basket test at 100 rpm in line with European Pharmacopoeia, Ph. Eur. 2.9.3. The MR formulation enables continuous nicotinamide release over a time period of 8 hours.

The MR capsules and IR pellets were then tested in dosages of 1000mg in 24 healthy subjects undergoing a randomized, open-label, single dose, 2-treatment 4-period, cross-over study in fasting and fed state (35). In the study, the modified release capsules showed significantly lower bioavailability and less frequently treatment emergent adverse events in comparison to an immediate release formulation.

Figure 1 shows the results of the bioavailability study of immediate release nicotinamide (R1, circles) versus modified release nicotinamide (T1, triangles). Fig. 1 shows the concentration time curve of nicotinamide serum levels after intake of singles doses of 1000 mg immediate release nicotinamide (R1) and 1000 mg modified release nicotinamide (T1). Modified release (T1) nicotinamide formulation reveals significantly lower bioavailability of nicotinamide with an AUC (area under curve) of 28% for the modified release formulation compared to IR formulation (fasted state); the Cₘₐₓ was 20% for the modified release formulation compared to the IR formulation (fasted state); Tₘₐₓ was achieved with 3.75 hours for the modified release formulation versus 0.50 hours for the IR formulation (R1).

Further results of the study are shown in Table 4.

**Table 4: Overview of safety results of immediate release nicotinamide (reference) versus modified release nicotinamide (test) in dosages of 1000mg in 24 healthy subjects (35); overall number of adverse events as well as events considered reasonably related to study medication were twice as high for the immediate release formulation compared to the modified release formulation.**

| **System organ class** | **Preferred term** | **Test 1 (T1) (fasted) N=24** | **Test 2 (T2) (fed) N=24** | **Reference 1 (R1) (fasted) N=24** | **Reference 2 (R2) (fed) N=24** |
|---|---|---|---|---|---|
| | | n (%) e | n (%) e | n (%) e | n (%) e |
| | Overall summary | 6 (25.0) 7 | 2 (8.3) 2 | 10 (41.7) 12 | 5 (20.8) 5 |
| Nervous system disorders | Summary data | 6 (25.0) 6 | 1 (4.2) 1 | 10 (41.7) 11 | 4 (16.7) 4 |
| | Headache | **6 (25.0) 6^{a}** | 1 (4.2) 1 | **10 (41.7) 11^{b}** | 4 (16.7) 4 |
| Gastrointestinal disorders | Summary data | 1 (4.2) 1 | - | 1 (4.2) 1 | - |
| | Nausea | - | - | 1 (4.2) 1 | - |
| | Toothache | 1 (4.2) 1 | - | - | - |
| Infections and infestations | Summary data | - | 1 (4.2) 1 | - | - |
| | Nasopharyngitis | - | 1 (4.2) 1 | - | - |
| Musculoskeletal and connective tissue disorders | Summary data | - | - | - | 1 (4.2) 1 |
| | Pain in extremity | - | - | - | 1 (4.2) 1 |
| n = number of subjects having the event, e = number of events (all post-dose events considered), | | | | | |
| (%) = proportion of exposed subjects having the event | | | | | |
| **bold** = events considered reasonably related →a = 3 events, b = 4 events considered reasonably related | | | | | |
| Treatment T1: Nicotinamide 4 x 250 mg modified release capsules - fasting | | | | | |
| Treatment T2: Nicotinamide 4 x 250 mg modified release capsules - fed | | | | | |
| Treatment R1: Nicotinsäureamid 5 x 200 mg JENAPHARM^{®} tablets - fasting | | | | | |
| Treatment R2: Nicotinsäureamid 5 x 200 mg JENAPHARM^{®} tablets - fed | | | | | |

Lower AUC, as well as the observed differences in Cₘₐₓ, Tₘₐₓ and other pharmacokinetic parameters for modified release formulations compared to immediate release formulation per se is not an unexpected finding and can be seen in many modified release formulations. However, these differences in pharmacokinetics normally lead to a differing clinical profile. Yet, with the present 250 mg modified release capsules, comparable or even better clinical efficacy in terms of efficacy for lowering serum phosphate levels was shown.

### Examples 1-2 and 1-3: Preparation of further pharmaceutical preparations containing modified release nicotinamide

Modified release (MR) nicotinamide capsules containing modified release nicotinamide pellets were prepared in the same way as in Example 1.1, unless noted otherwise, using the following ingredients (with the approximate mass given per capsule).

### Example 1-2

The pellets of Example 1-2 were produced in the same way as in Example 1-1 (except using talc instead of glycerol monostearate), with the following amounts of the components: nicotinamide (250.00 mg), microcrystalline cellulose (107.14 mg), ethyl cellulose (18.00 mg), dibutyl sebacate (1.29 mg), hydroxypropyl methylcellulose (1.29 mg), talc (5.14 mg), talc (0.89 mg), colloidal anhydrous silica (0.89 mg).

The pharmaceutical preparation obtained in Example 1-3 showed a release of nicotinamide of about 25 - 55 % by weight from the pharmaceutical preparation at a pH of about 1.0 at a time period of 2 hours and of at least about 70 % by weight from the pharmaceutical preparation after dissolution at a pH of about 1.0 for a time of about 2 hours and subsequent dissolution at a pH of about 6.8 for about 4 hours, as measured with the basket test described with regard to Fig. 3.

### Example 1-3:

In contrast to Example 1-1 two types of pellets (immediate release and modified release, e.g. extended release, pellets were combined in one capsule. The uncoated IR pellets are produced analogously to Example 1-1, but the extended release pellets are coated using an aqueous coating method.

The following components were used:
nicotinamide (190.000 mg), microcrystalline cellulose (81.43 mg), ethyl cellulose (14.18 mg), ammonium hydroxide (0.93 mg), dibutyl sebacate (3.02 mg), oleic acid (1.66 mg), colloidal anhydrous silica (1.51 mg), talc (0.37 mg), colloidal anhydrous silica (0.37 mg).

The pharmaceutical preparation obtained in Example 1-3 showed a release of nicotinamide of about 25 - 55 % by weight from the pharmaceutical preparation at a pH of about 1.0 at a time period of 2 hours and of at least about 70 % by weight from the pharmaceutical preparation after dissolution at a pH of about 1.0 for a time of about 2 hours and subsequent dissolution at a pH of about 6.8 for about 4 hours, as measured with the basket test described with regard to Fig. 3.

### Example 2: Phase II dose finding and proof of concept study (36)

As modified release nicotinamide capsules with a dosage of 250 mg, the ones produced in Example 1-1 were used in Example 2. For higher dosages, multiple capsules were applied, i.e. two, three or four for dosages of 500 mg, 750 mg and 1000 mg.

For a comparative example in this study, immediate release (IR) pellets with 250 mg nicotinamide were produced by mixing with microcrystalline cellulose (70 wt.% nicotinamide and 30 wt.% microcrystalline cellulose) and purified water in amounts of 105.0 kg nicotinamide, 45.0 kg microcrystalline cellulose, and 37.5 kg purified water for a 150 kg batch of IR pellets, followed by granulation, extrusion, spheronization, drying and classification.

In this randomized study 252 patients on hemodialysis were studied for the treatment with nicotinamide for up to 8 weeks. In a double blind setting modified release nicotinamide (MR) was given at daily doses of 250 mg, 500 mg, 750 mg and 1,000 mg. Additionally safety and efficacy of 1,000 mg immediate release nicotinamide (IR) produced in Example 2 was investigated. One thousand mg nicotinamide modified release capsules were given once daily while immediate release tablets were given in three dosages per day (250 mg - 500 mg - 250 mg p.o.).

The main question of this trial was whether there was a dose response dependency for nicotinamide modified release capsules based on serum phosphate concentration values 4 weeks post randomization. Secondary outcome criteria included a comparison of the two different galenics of nicotinamide by means of several measures of safety and efficacy.

The confirmatory analysis showed a significant (p<0.0001) quadratic dose response shape in reduction of the phosphate level. To achieve an effect of a 0.22603 mmol/l phosphate level reduction against an independent baseline group (the values of the 1000 mg IR were used, called compare group), a minimum effective dose of 450 mg of nicotinamide was needed.

Concerning secondary endpoints, for all phosphate linked efficacy measures a significant treatment effect was observed. Concerning the comparison of the two different galenics, this example revealed heterogeneous results concerning predefined efficacy endpoints. Secondary outcome measures revealed similar clinical efficacy concerning mean reduction of serum phosphate levels for the modified and immediate release formulations over the 8 week lasting treatment period, as seen in Figure 2.

Figure 2 therein shows exemplary immediate release nicotinamide 1,000mg per day (IR-NA = immediate release nicotinamide, solid line) given in three dosages per day (250mg-500mg-250mg p.o.) versus modified release nicotinamide 1,000mg per day (0mg-1000mg-0mg p.o.; MR-NA = modified release nicotinamide, dashed line), and revealed similar efficacy concerning mean reduction of serum phosphate levels over the 8-week lasting treatment period.

Other secondary outcome measures are summarized in the Table 5 below.

**Table 5: Predefined secondary outcome measures of the example at the end of treatment. Concerning the two 1,000 mg dosing groups the modified release formulation (MR) showed comparable or even better results than the immediate release formulation (IR). Secondary outcome measures with group differences > 15% are highlighted.**

| Secondary Endpoint | 250mg MR | 500mg MR | 750mg MR | 1000mg MR | 1000mg IR |
|---|---|---|---|---|---|
| Mean serum PO₄ at week 8 (ITT; mmol/L) | 1.994 ± 0.33 | 1.948 ± 0.45 | 1.886 ± 0.50 | 1.849 ± 0.52 | 1.864 ± 0.40 |
| Mean PO₄, difference to baseline at week 8 (ITT; mmol/L) | -0.080 ± 0.33 | -0.126 ± 0.38 | -0.145 ± 0.43 | **-0.323 ± 0.48** | **-0.286 ± 0.41** |
| Proportion of ITT-patients with PO₄ < 1.52 mmol/L at week 8 | 3% | 17% | 15% | **25%** | **21%** |
| Proportion of ITT-patients with PO₄ < 1.78 mmol/L at week 8 | 24% | 34% | 47% | 47% | 46% |
| Proportion of ITT-patients with PO₄ decrease from baseline to week 8 ≥ 20% | 14% | 20% | 21% | 39% | 24% |
| ITT = intention to treat, PO₄ = phosphate, MR = modified release formulation, IR = immediate release formulation. | | | | | |

While mean serum phosphate levels revealed no differences between the immediate and modified release formulation, difference to baseline of serum phosphate levels as well as the proportion of patients that fulfilled the responder criterions (serum phosphate levels < 1.78 mmol/L and < 1.52 mmol/L) exhibited group differences of more than 15% each with an advantage for the modified release formulation.

### Clinical safety and tolerability.

Trial discontinuations due to adverse events increased with the amount of daily nicotinamide intake from 16.7% (250 mg/d MR) to 24.5% (1.000 mg/d MR) of patients. The highest number of adverse event related trial discontinuations was observed after treatment with the immediate release formulation (n=17 (33.3%)), see Table 6.

**Table 6: Summary of study discontinuations and adverse events over the different DONATO (36) treatment groups. Under treatment with IR nicotinamide more patients discontinued treatment because of adverse events and more adverse events were assessed as drug related.**

| | 250mg MR | 500mg MR | 750mg MR | 1000mg MR | 1000mg IR |
|---|---|---|---|---|---|
| N (ITT) | 48 | 50 | 50 | 53 | 51 |
| Overall Study discontinuation | 13 | 15 | 16 | 19 | 19 |
| Study discontinuation due to AE | 8 | 9 | 9 | **13** | **17** |
| Overall number of AE | 71 | 64 | 101 | **138** | **111** |
| AE assessed as related, probably related or possibly related to study medication | 36.6 % | 26.7 % | 38.7 % | **31.8 %** | **44.1 %** |
| Patients with SAE | 4 | 5 | 2 | 7 | 5 |
| Fatal AE | 1 | | | | 1 |
| ITT = Intention to treat group, MR = modified release nicotinamide, IR = immediate release nicotinamide, AE = adverse event, SAE = serious adverse event. | | | | | |

Under investigational treatment 485 adverse events were reported in 182 out of 252 patients (72%). Adverse events assessed as possibly related, probably related or related to study medication ranged between 26.7% and 38.7% for the modified release formulations. This number was higher in the treatment group 1000 mg IR (44.1%). Direct comparison of the two 1000 mg treatment groups revealed a nearly 40% increase of treatment related AE under treatment with IR nicotinamide compared to the MR formulation. Accordingly, study discontinuations due to AE were 31% higher for the IR formulation compared to the 1000 mg MR treatment group (17 versus 13 patients, see table 6).

In conclusion, this example revealed at least comparable and for several predefined secondary endpoints even better efficacy of the modified release formulation compared to the immediate release formulation. Despite better efficacy, under modified release treatment fewer patients experienced drug related side effects and fewer patients discontinued treatment due to adverse events.

In this example, also plasma levels of nicotinamide were measured under steady state conditions in dialysis patients. Analogous to the pharmacokinetic trial, bioavailability of the MR formulation was considerably lower compared to the IR formulation, as can be seen in Table 7. This is in line with the better safety profile of the modified release formulation observed in both trials. In contrast, the comparable or even better efficacy in the reduction of elevated plasma phosphate levels of the MR formulation was really unexpected.

The unexpected differences in pharmacodynamic action correspond with differences in the metabolism of both formulations. Beneath the parent drug, also plasma levels of nicotinamide adenine dinucleotide (NAD) and N-methylnicotinamide were measured. NAD is the metabolite essential for most physiological actions of nicotinamide (see e.g. Yang, 2004 (37)), and N-methylnicotinamide is also linked to specific physiological functions in comparison to nicotinamide (see e.g. Mogielnicki, 2007 (38)). Despite the considerably lower bioavailability of the parent drug under treatment with the MR formulation, plasma levels of both metabolites were 45-50% higher compared to treatment with the IR formulation, see Tables 8 and 9). Thus differences in the risk-benefit-ratio of the two formulations might be based on differences in the metabolism resulting in formulation specific patterns of metabolite bioavailability.

**Table 7: Plasma levels of Nicotinamide at baseline and after 8 weeks of treatment. Under treatment with daily 1000 mg modified release nicotinamide (MR) plasma levels were three times lover compared to the 1000 mg immediate release formulation (IR).**

| **Nicotinamide in µg/L in the Safety Population** | | | | | | |
|---|---|---|---|---|---|---|
| **Visit** | **Medication** | **N** | **Mean** | **SD** | **SEM** | **p-value*** |
| W0 | 250mg MR | 47 | 21,91 | 19,457 | 2,838 | |
| W0 | 500mg MR | 50 | 20,02 | 11,876 | 1,68 | |
| W0 | 750mg MR | 50 | 18,13 | 11,127 | 1,574 | |
| W0 | 1000mg MR | 53 | 25,18 | 45,801 | 6,291 | |
| W0 | 1000mg IR | 50 | 23,92 | 23,083 | 3,264 | |
| W0 | All | 250 | 21,87 | 25,916 | 1,639 | 0,7272 |
| W8 | 250mg MR | 38 | 27,61 | 36,324 | 5,892 | |
| W8 | 500mg MR | 41 | 65,75 | 243,486 | 38,026 | |
| W8 | 750mg MR | 36 | 46,51 | 107,541 | 17,923 | |
| **W8** | **1000mg MR** | **42** | **161,36** | **452,711** | **69,855** | |
| **W8** | **1000mg IR** | **34** | **508,2** | **1152,8** | **197,703** | |
| W8 | All | 191 | 154,32 | 565,188 | 40,896 | **0,0014** |
| *p-value of Kruskal-Wallis test for Treatment groups | | | | | | |

**Table 8: Plasma levels of the Nicotinamide metabolite nicotinamide adenine dinucleotide (NAD) at baseline and after 8 weeks of treatment. Under treatment with daily 1000 mg modified release nicotinamide (MR) plasma levels were 45% higher compared to the 1000 mg immediate release formulation (IR).**

| **Nicotinamide Adenine Dinucleotide (NAD) in mg/L in the Safety Population** | | | | | | |
|---|---|---|---|---|---|---|
| **Visit** | **Medication** | **N** | **Mean** | **SD** | **SEM** | **p-value*** |
| W0 | 250mg MR | 47 | 10,99 | 8,092 | 1,18 | |
| W0 | 500mg MR | 50 | 10,2 | 6,986 | 0,988 | |
| W0 | 750mg MR | 50 | 9,44 | 7,414 | 1,048 | |
| W0 | 1000mg MR | 53 | 10,04 | 7,623 | 1,047 | |
| W0 | 1000mg IR | 50 | 10,99 | 10,468 | 1,48 | |
| W0 | All | 250 | 10,32 | 8,159 | 0,516 | 0,6813 |
| W8 | 250mg MR | 38 | 13,27 | 11,637 | 1,888 | |
| W8 | 500mg MR | 41 | 15,26 | 11,364 | 1,775 | |
| W8 | 750mg MR | 37 | 21,73 | 24,26 | 3,988 | |
| **W8** | **1000mg MR** | **42** | **24,22** | **17,951** | **2,77** | |
| **W8** | **1000mg IR** | **35** | **16,67** | **14,232** | **2,406** | |
| W8 | All | 193 | 18,31 | 16,895 | 1,216 | **0,0169** |
| *p-value of Kruskal-Wallis test for Treatment groups | | | | | | |

**Table 9: Plasma levels of the Nicotinamide metabolite N-methylnicotinamide at baseline and after 8 weeks of treatment. Under treatment with daily 1000 mg modified release nicotinamide (MR) plasma levels were 49% higher compared to the 1000 mg immediate release formulation (IR).**

| **N-Methylnicotinamide in µg/L in the Safety Population** | | | | | | |
|---|---|---|---|---|---|---|
| **Visit** | **Medication** | **N** | **Mean** | **SD** | **SEM** | **p-value*** |
| W0 | 250mg MR | 47 | 31,2 | 26,688 | 3,893 | |
| W0 | 500mg MR | 50 | 25,34 | 30,891 | 4,369 | |
| W0 | 750mg MR | 50 | 24,68 | 18,455 | 2,61 | |
| W0 | 1000mg MR | 53 | 33,59 | 37,206 | 5,111 | |
| W0 | 1000mg IR | 50 | 34,32 | 67,617 | 9,562 | |
| W0 | All | 250 | 29,85 | 39,9 | 2,523 | 0,5273 |
| W8 | 250mg MR | 38 | 87,86 | 74,373 | 12,065 | |
| W8 | 500mg MR | 41 | 121,4 | 113,949 | 17,796 | |
| W8 | 750mg MR | 36 | 243,29 | 213,369 | 35,562 | |
| **W8** | **1000mg MR** | **42** | **597,67** | **608,607** | **93,91** | |
| **W8** | **1000mg IR** | **34** | **401,39** | **493,128** | **84,571** | |
| W8 | All | 191 | 292,27 | 414,995 | 30,028 | **<.0001** |
| *p-value of Kruskal-Wallis test for Treatment groups | | | | | | |

### Example 3:

The combination therapy approach is especially promising in the treatment of hyperphosphatemia because the pharmacological target of nicotinamide, the cotransporter NaPi2b is strongly regulated up under treatment with phosphate binders. In this regard it is also again noted that with modified release nicotinamide a phosphate binder that negatively affects the nicotinamide uptake, e.g. like sevelamer and/or a derivative thereof, can alleviate these negative effects of such phosphate binder when given concomitantly.

Two different mechanisms account for intestinal phosphate absorption. Under conditions of normal dietary phosphate availability, passive paracellular diffusion accounts for the majority of phosphate uptake. In case of dietary phosphate restriction or under treatment with phosphate binders that prevent intestinal phosphate uptake, the active cotransporter driven absorption process is raised resulting in enhanced bioavailability of phosphate from food. To investigate the contribution of NaPi2b cotransporter to intestinal phosphate absorption in kidney disease, kidney injury was induced by adenine treatment in wild type (WT) or conditional NaPi2b knockout mice (NaPi-KO) (Schiavi 2012 (39)). Fig. 4a and 4b show results of this investigation. Fig. 4a,b from: Schiavi 2012 (39), shows the quantification of the relative sodium-phosphate cotransporter 2b (NaPi2b) expression compared to ß-actin protein and demonstrates a decrease in CKD (Adenine) compared to controls (Chow) and significant increase in CKD animals under phosphate binder treatment (Adenine + Sevelamer). No expression of NaPi2b was observed in NaPi2b knockout mice (NaPi-KO). # p<0.05 versus Adenine. Fig. 4b, adapted from: Schiavi 2012 (39), shows serum Phosphate (Pi) balance in wild type (WT) and NaPi2b knockout mice (NaPi-KO). Pi was significantly elevated in uremic (Adenine) WT mice. This effect was attenuated in uremic NaPi-KO mice. Phosphate binder treatment (Adenine + Sevelamer) normalized Pi in NaPi-KO mice while elevated Pi levels remained unaffected in WT mice, indicating that phosphate binder treatment was counteracted by compensatory regulation of the NaPi2b cotransporter.

In WT mice, adenine treatment reduced intestinal NaPi2b protein expression by 50% while these animals experienced a 6-fold increase of the cotransporter under phosphate binder treatment (Fig. 4a), while the cotransporter protein was not detectable in NaPi-KO mice. Adenine treatment generally resulted in hyperphosphatemia although phosphate levels in WT mice were significantly higher than in NaPi-KO mice (Fig. 4b). In WT mice, phosphate binder treatment did not affect hyperphosphatemia while NaPi-KO mice were normophosphatemic under binder treatment (Fig 4b) indicating that in WT mice the upregulation of NaPi2b completely counteracted the lower phosphate availability due to binder treatment. Phosphate binder triggered upregulation of NaPi2b is completely abolished under combination therapy with nicotinamide.

Further, the effect of nicotinamide treatment on intestinal NaPi2b protein expression in a mouse model (DBA/2 mouse) was investigated.

For this purpose, 5/6 nephrectomized DBA/2 mice were used as model for vascular calcification. The mice were treated with nicotinamide (NA) in a concentration of 600 µg/mL in the drinking water.

The treatment only resulted in a decrease of S-phosphate in healthy DBA/2 mice with high phosphate diet (phosphorus content 1.03 % (w/w)); composition of basic food [% (w/w)]: dry substance: 87.7; crude protein (N x 6,25) 19; crude fat 3.3; crude fiber 4.9; crude ash 6.4; N-free extractives 54.1; starch 36.5; sugar 4.7; calcium 0.9; phosphate 1.03; magnesium 0.22). NA, the phosphate binder magnesium (Mg) and the NA+Mg combination therapy resulted in a reduction of fractional phosphate elimination und reduction of FGF23. NA did not show any influence on NaPi2b-RNA and corresponding cotransporter content of the small intestine. Mg resulted in a notable increase of NaPi2b-cotransporter, whereas NA + Mg lead again to a decrease.

In this model induction of kidney disease resulted in a reduction of NaPi2b expression by more than 50% while treatment with the phosphate binder magnesium carbonate for 7 weeks resulted in a 10-fold increase of cotransporter expression (Fig. 5). Combined treatment of phosphate binder and nicotinamide completely restored cotransporter upregulation indicating that NaPi2b inhibition is especially useful under phosphate binder treatment.

Figure 5 shows NaPi2b immune fluorescence in a mouse model of chronic kidney disease (CKD). Treatment with the phosphate binder magnesium carbonate strongly enhances intestinal NaPi2b protein density (CKD Mg) while combined treatment of nicotinamide and phosphate binder (CKD NA Mg) completely prevents NaPi2b overexpression. In Fig. 5 the quantification of intestinal (ileum) expression of NaPi2b protein is shown. Given is the amount and standard deviation of NaPi2b protein immune fluorescence in control DBA/2 mice treated with nicotinamide (ctrl + NA), 5/6 nephrectomized mice (CKD), nicotinamide treated CKD mice (CKD + NA), nicotinamide and phosphate binder (magnesium carbonate) treated CKD mice (CKD + NA + Mg) and phosphate binder treated CKD mice (CKD + Mg). CKD resulted in slightly reduced expression of NaPi2b (CKD) while treating CKD mice with phosphate binder strongly increased NaPi2b protein (CKD + Mg). This upregulation was completely abolished under add-on-treatment with nicotinamide (CKD + NA + Mg). Bars between columns indicate significant between groups differences (ANOVA with Tukey Post Test, p<0.05).

Thus the new investigation strongly suggests that nicotinamide treatment for lowering of phosphate burden is especially effective in combination therapy with therapeutic approaches intended for the restriction of intestinal phosphate availability, namely dietary phosphate restriction as well as oral phosphate binder treatment by
(1) Resolving the physiological compensatory upregulation of intestinal phosphate availability that restricts the therapeutic efficacy of existing treatment options for hyperphosphatemia.
(2) Enhancing the therapeutic gain of nicotinamide treatment in situations when its pharmacological target, the NaPi2b cotransporter, is regulated up.

### Nicotinamide for the prevention of hyperphosphatemia by enhancement of renal expression of the sodium-phosphate cotransporter NaPi2b in patients with CKD stages 1-5 and residual kidney function.

The newly observed physiological interactions of combined phosphate binder and nicotinamide treatment on the expression of phosphate cotransporter proteins in the intestine indicate that this new intervention should be useful as a pharmacological intervention to treat hyperphosphatemia both in patients with CKD as well as in patients with end stage renal disease. The preclinical investigation of nicotinamide action in the DBA/2 mouse model for CKD also revealed for the first time a new second mode of action that might reduce phosphate burden especially in patients with moderate kidney disease with residual kidney function.

According to Table 2, the kidneys express three different phosphate cotransporters (NaPi2a, NaPi2c, PiT2). They are located in the proximal part of the tubule apparatus, at the apical side of kidney epithelial cells (Forster, 2013 (56)). Their physiological role is the reabsorption of filtrated phosphate from the primary urine. Recently, the phosphate cotransporter NaPi2b was detected in the kidney of rats (Suyama, 2012 (11)). In contrast to the cotransporters mentioned above, NaPi2b is expressed at the basolateral side of epithelial cells surrounding the urinary duct and it was suggested that the physiological role is to enhance basal phosphate excretion levels in the kidney. In line with this assumption, renal NaPi2b expression is strongly enhanced under high phosphorus diet (Suyama, 2012 (11)). Moreover, in a mouse model of adenine induced CKD, renal expression of NaPi2b was also significantly enhanced, while expression of NaPi2a and NaPi2c was reduced (Pulskens, 2015, (57)).

The present inventors explored the effect of nicotinamide treatment on renal NaPi2b cotransporter expression in 5/6 nephrectomized DBA/2 mice. As already shown (Pulskens, 2015 (57)), CKD induced an enhancement of renal NaPi2b RNA as well as renal protein expression. In contrast, nicotinamide treatment resulted in a strong and significant enhancement of renal NaPi2b expression, as shown in Figure 6.

Figure 6 shows therein the quantification of renal expression of NaPi2b protein. Shown are the amount and standard deviation of NaPi2b protein immune fluorescence in control DBA/2 mice treated with nicotinamide (ctrl + NA), 5/6 nephrectomized mice (CKD), nicotinamide treated CKD mice (CKD + NA), mice treated with the phosphate binder magnesium carbonate (CKD + Mg) and animals treated both with nicotinamide and phosphate binder (CKD + NA + Mg). CKD resulted in slightly enhanced expression of NaPi2b in the remaining kidney tissue. Treatment of CKD mice with nicotinamide strongly increased NaPi2b protein signal. Moreover, NaPi2b cotransporter was additionally increased under combination treatment with the phosphate binder while treatment with the phosphate binder alone showed no significant difference compared to baseline. Bars between columns indicate significant between groups differences (ANOVA with Tukey Post Test, p<0.05).

In this animal model of moderate CKD it thus could be shown for the first time that nicotinamide provokes a dual mode of action with reduction of NaPi2b expression in the intestine (Figure 5) as well as enhancement of NaPi2b expression in the kidney. Reduced intestinal NaPi2b expression reduces intestinal phosphate uptake and enhanced renal NaPi2b expression enhances renal fractional phosphate excretion. Thus both pharmacological actions synergistically reduce systemic phosphate load in CKD. The current experimental data indicate that nicotinamide is especially effective in prevention and treatment of hyperphosphatemia in moderate kidney disease.

### Example 4:

For further testing adverse effects of treatment with modified release nicotinamide, a literature search was done regarding side effects of immediate release nicotinamide, particularly flush and thrombocytopenia.

Table 10 shows literature considered.

**Table 10: Literature on immediate release nicotinamide treatment of hyperphosphatemia.**

| Study Reference | Patient number | Patients | Nicotinamide dose (mg/d) | Treatment Duration | Treatment discontinuations due to thrombocytopenia and flushing | Side effects thrombocytopenia and flushing |
|---|---|---|---|---|---|---|
| Takahashi et al. 2004 (19) | 65 | Hemodialysis | 500-1500 | 12 weeks | Thrombocytopenia 1 | Thrombocytopenia 1 |
| Galeano et al. 2005 (40) | 12 | Hemodialysis | 1000 | 8 weeks | Thrombocytopenia 1 | Thrombocytopenia 1 |
| Rahmouni et al. 2005 (20) | 10 | Not reported | 500-1000 | 8 weeks | - | - |
| Rottembourg et al. 2005 (41) | 6 | Hemodialysis | 1000 | Not specified | Thrombocytopenia 5 | Thrombocytopenia 5 |
| Delanaye et al. 2006 (42) | 6 | Hemodialysis | Not specified | Not specified | - | - |
| Olivero et al. 2006 (43) | 33 | Hemodialysis | 750/1000 | 12 weeks | - | - |
| Cheng et al. 2008 (21) | 33 | Hemodialysis | 500-1500 | 8 weeks | - | Thrombocytopenia 1 |
| del Carmen Reinoso et al. 2009 (44) | 32 | Hemodialysis | Up to 1500 | ≥ 12 weeks | - | - |
| Young et al. 2009 (22) | 8 | Peritoneal dialysis | 500-1500 | 8 weeks | - | - |
| Shahbazian et al. 2011(45) | 24 | Hemodialysis | 500-1000 | 8 weeks | - | Thrombocytopenia 8 |
| Vasantha et al. 2011 (46) | 30 | Hemodialysis | 500-750 | 8 weeks | - | - |
| Allam et al. 2012 (47) | 30 | Hemodialysis | 500-1000 | 8 weeks | Flushing 2 | Flushing 2 |
| Lenglet et al. 2016 (48) | 49 | Hemodialysis | 500-2000 | 24 weeks | Thrombocytopenia 4 | Thrombocytopenia 4 |
| El Borolossy et al. 2016 (49) | 30 | Children on hemodialysis | 200-300 | 24 weeks | - | Flushing 8 |
| Overall | 368 | | | | **Thrombocytopenia 3,0% (n=11) Flushing 0,5% (n=2)** | **Thrombocytopenia 5,4% (n=20) Flushing 2,7% (n=10)** |

From the literature review, in a total of 368 patients 5,4% showed thrombocytopenia und 2,7% flush. In contrast, these side effects were not observed with the present modified release nicotinamide used in the Examples.

The inventors concluded that once daily intake of modified release nicotinamide in daily doses of up to 1500 mg is effective in lowering elevated blood phosphate levels. Modified release nicotinamide is more effective and triggers less adverse drug reactions compared to thrice daily intake of immediate release nicotinamide. The better risk-benefit-ratio is linked to specific differences in the metabolism of modified and immediate release nicotinamide in humans. As shown in Tables 7 to 9, the DONATO trial (36) revealed lower blood levels of the parent drug under treatment with the modified release formulation while blood levels of several pharmacologically active metabolites were increased compared to the immediate release formulation.

A better risk-benefit-ratio in the treatment of hyperphosphatemia in hemodialysis patients was also described for extended release nicotinic acid (Sampathkumar, 2006 (50)). However, this finding does not predict a comparable action of modified release nicotinamide, as both drugs are clearly distinguished as different chemical entities. Although both drugs are traditionally denominated as vitamin B3, the conversion between the two chemicals is under enzymatic control, and nicotinamide is not converted to nicotinic acid in man (see e.g. Gillmor, 1999 (51); Petley, 1995 (52)). Both drugs differ considerably concerning pharmacological action (see e.g. Niacinamide Monograph, 2002 (53)). Moreover, the safety profiles of the two drugs are essentially different with tolerable upper intake levels of 10 mg/day for nicotinic acid and 900 mg/day for nicotinamide (see e.g. Scientific Committee on Food, 2002 (54)).

### Example 5

Furthermore, most phosphate binders do not specifically react with phosphate but also bind other polar small molecules in the gut and thereby may inhibit their intestinal absorption (see e.g. Neradova, 2016 (55)). In fact, nicotinamide is ineffective in the treatment of hyperphosphatemia when given in combination with the phosphate binder sevelamer (see Olivero, 2006 (43)), which is known for its broad intestinal interaction potential. The summary of product characteristics for sevelamer carbonate (Sevemed®) states the following: "Sevemed is not absorbed and may affect the bioavailability of other medicinal products. When administering any medicinal product where a reduction in the bioavailability could have a clinically significant effect on safety or efficacy, the medicinal product should be administered at least one hour before or three hours after Sevemed, or the physician should consider monitoring blood levels." Due to their mode of action phosphate binders must be taken three times daily with meals and thus consequently interact with immediate release nicotinamide, which must be taken also twice or three times daily. As the modified release formulation of nicotinamide enables once daily intake of nicotinamide at night time for the treatment of hyperphosphatemia, it can be efficient in a combination therapy approach together with phosphate binders. The modified release formulation releases nicotinamide over a time period of at least 8 hours (see e.g. figure 3), enabling drug release over the whole night, and thus minimizes risk of interaction with phosphate binders. This is very different from immediate release nicotinamide formulations where peak plasma levels were observed within 30 minutes after ingestion (see figure 1). Thus, even if the immediate release formulation would be given at nighttime, the potential for interaction with prior swallowed phosphate binders would be considerably higher.

Example 6:

### Nicotinamide for the dual improvement of serum phosphate as well as Lp(a) levels in patients with CKD.

In order to investigate the possible effect of nicotinamide on the lowering of raised serum levels of Lp(a), the present inventors undertook a preclinical study in a transgenic mouse model. Lp(a) is biosynthesized only in humans and old world monkeys which complicates the use of animal models to investigate Lp(a) metabolism directly (Kostner, 2013 (72)). Lp(a) consists of LDL covalently bound to Apo(a). Plasma levels of Lp(a) highly correlate with Apo(a) synthesis in man. As Lp(a) synthesis is limited to primates, a transgenic mouse model was used where the entire Apo(a) gene including the promotor region was introduced to the mouse genome (Chennamsetty, 2012 (31)). In the transgenic Apo(a) mice (tg-Apo(a)) Apo(a) is expressed mainly in female mice. These female mice were shown to exhibit a 43% reduction in plasma Apo(a) protein as well as a 65% reduction in Apo(a) mRNA transcript in liver cells in response to oral treatment with 1% nicotinic acid in the chow (Chennamsetty, 2012 (31)).

From this, a model of the supposed mode of action of nicotinic acid in reduction of Lp(a) was prepared, as shown in Figure 7. Nicotinic acid (niacin) binds to the specific nicotinic acid receptor GPR109A. GPR109A inhibits adenylatcyclase responsible for the synthesis of adenosine-triphosphate (ATP) to cyclic adenosine-monophosphate (cAMP). Lower concentrations of cAMP reduce the activation of nuclear cAMP response elements (cAMP-RE) and thus reduces transcription of the Apo(a) gene (APOA).

To investigate the effect of nicotinamide on plasma Apo(a) levels, heterozygote tg-Apo(a) female mice received either 1% nicotinamide or 1% nicotinic acid orally as food supplements in a cross-over design for two weeks. Levels of Apo(a) were determined by means of ELISA and were expressed as Lp(a) in mg/dl as each molecule Apo(a) binds one molecule of LDL to form Lp(a). Nicotinamide treatment resulted in a 67% reduction of Lp(a) after week 1 and a 77% reduction after two weeks of treatment compared to baseline levels (p<0,0001; t-Test and Wilcox), as also shown in Fig. 8. Figure 8 shows the reduction of serum levels Lp(a) in transgenic Apo(a) female mice treated with standard chow (Maintenance Diet, Altromin Spezialfutter GmbH & Co. KG, Germany: crude protein 191970,400 [mg/kg]; crude fat 40803.010 [mg/kg]; crude fiber 60518.480 [mg/kg]; crude ash 69364.890 [mg/kg]; moisture 112946.890 [mg/kg]; disaccharide(s) 49464.050 [mg/kg]; polysaccharides 358852.330 [mg/kg]; metab. energy 3188.487 [kcal/kg]; lysine 8026.060 [mg/kg]; methionine 2738.230 [mg/kg]; cysteine 3171.100 [mg/kg]; threonine 6611.330 [mg/kg]; tryptophan 2458.450 [mg/kg]; arginine 11503.050 [mg/kg]; histidine 4465.100 [mg/kg]; isoleucine 7560.450 [mg/kg]; leucine 13416.500 [mg/kg]; phenylalanine 8326.500 [mg/kg]; valine 8858.100 [mg/kg]; alanine 8557.750 [mg/kg]; aspartic acid 15905.350 [mg/kg]; glutamic acid 38495.600 [mg/kg]; glycine 8345.100 [mg/kg]; proline 12427.300 [mg/kg]; serine 9127.550 [mg/kg]; tyrosine 5962.050 [mg/kg]; vitamin A 15000.000 [I.E./kg]; vitamin D3 600.000 [I.E./kg]; vitamin E 110.350 [mg/kg]; vitamin K3 as menadione 3.000 [mg/kg]; vitamin B1 18.000 [mg/kg]; vitamin B2 12.000 [mg/kg]; vitamin B6 9.000 [mg/kg]; vitamin B12 0.024 [mg/kg]; nicotinic acid 36.000 [mg/kg]; panthothenic acid 21.000 [mg/kg]; folic acid 2.33500 [mg/kg]; biotin 0.250 [mg/kg]; choline chloride 699.000 [mg/kg]; vitamin C 36.000 [mg/kg]; calcium 7114.940 [mg/kg]; phosphorus 5090.560 [mg/kg]; digest. phosphorus 1537.500 [mg/kg]; magnesium 2436.930 [mg/kg]; sodium 2156.565 [mg/kg]; potassium 9214.900 [mg/kg]; sulfur 1198.200 [mg/kg]; chlorine 3541.000 [mg/kg]; iron 198.037 [mg/kg]; manganese 97.686 [mg/kg]; zinc 94.876 [mg/kg]; copper 13.582 [mg/kg]; iodine 1.623 [mg/kg]; molybdenum 1.129 [mg/kg]; fluorine 2.192 [mg/kg]; selenium 0.265 [mg/kg]; cobalt 0.351 [mg/kg]; palmitic acid C-16:0 3581.475 [mg/kg]; stearic acid C-18:0 1094.300 [mg/kg]; oleic acid C-18:1 6292.225 [mg/kg]; linoleic acid C-18:2 2038.700 [mg/kg] linolenic acid C-18:3 2038.700 [mg/kg]; arachidic acid C-20:0 40.000 [mg/kg]; eicosaeic acid (Eicosaensäure) C-20:1 50.000 [mg/kg]; aluminum 97.963 [mg/kg]; volume 1000.000 [kg]) containing 1 wt.% nicotinic acid (A) or 1 wt.% nicotinamide (B), based on the whole composition. Treatment effect is shown for baseline (week 0) and after 1 and 2 weeks of treatment. Triple stars indicate significant reductions of Lp(a) compared to baseline (t-test).

Also treatment with nicotinic acid resulted in an decline of Lp(a) (week 1: 37%, p=0,011; week 2: 50%, p<0,001; see Fig. 8), although this reduction was less pronounced compared to nicotinamide. Reduction of Apo(a) and Lp(a) was in the same range as described earlier for tg-Apo(a) mice (Chennamsetty, 2012 (31)). In contrast, the Lp(a) lowering effect of nicotinamide was unexpected as this substance does not bind or otherwise affect the GPR109A receptor which was shown to mediate the pharmacological actions of nicotinic acid (Tunaru, 2005 (33)). In addition, the even stronger lipid lowering effects of nicotinamide point to a new pharmacological mode of action, independent from GPR109A binding.

As discussed above, high levels of Lp(a) trigger both the development and progression of CKD as well as elevated cardiovascular morbidity and mortality in patients with advanced CKD. As dyslipidemia and hyperphosphatemia on one hand both synergistically trigger development of cardiovascular calcifications (see above) and on the other hand progression of CKD is an independent risk factor for incidence and severity of hyperphosphatemia, lowering of elevated levels of Lp(a) might be beneficial in CKD patients both in terms of reducing the risk and frequency of hyperphosphatemic periods as well as lowering the risk for cardiovascular outcomes related to hyperphosphatemia. Thus nicotinamide reduces risk and strength of hyperphosphatemia in patients with CKD by dual action on (Kettler, 2011 (58)) the inhibition of the intestinal NaPi-2b cotransporter as well as by lowering serum levels of Lp(a). Moreover, as Lp(a) and hyperphosphatemia both promote cardiovascular calcification, both action may be beneficial in improvement of clinical outcomes.

### Literature

1. Young EW, Akiba T, Albert JM, McCarthy JT, Kerr PG, Mendelssohn DC, et al. Magnitude and impact of abnormal mineral metabolism in hemodialysis patients in the Dialysis Outcomes and Practice Patterns Study (DOPPS). Am J Kidney Dis. 2004;44(5 Suppl 2):34-8.
2. Tentori F, Blayney MJ, Albert JM, Gillespie BW, Kerr PG, Bommer J, et al. Mortality risk for dialysis patients with different levels of serum calcium, phosphorus, and PTH: the Dialysis Outcomes and Practice Patterns Study (DOPPS). Am J Kidney Dis. 2008;52(3):519-30.
3. Levin A, Bakris GL, Molitch M, Smulders M, Tian J, Williams LA, et al. Prevalence of abnormal serum vitamin D, PTH, calcium, and phosphorus in patients with chronic kidney disease: results of the study to evaluate early kidney disease. Kidney Int. 2007;71(1):31-8.
4. Saito H, Maeda A, Ohtomo S, Hirata M, Kusano K, Kato S, et al. Circulating FGF-23 is regulated by 1alpha,25-dihydroxyvitamin D3 and phosphorus in vivo. J Biol Chem. 2005;280(4):2543-9.
5. Block GA, Hulbert-Shearon TE, Levin NW, Port FK. Association of serum phosphorus and calcium x phosphate product with mortality risk in chronic hemodialysis patients: a national study. Am J Kidney Dis. 1998;31(4):607-17.
6. London GM, Guerin AP, Marchais SJ, Metivier F, Pannier B, Adda H. Arterial media calcification in end-stage renal disease: impact on all-cause and cardiovascular mortality. Nephrol Dial Transplant. 2003;18(9):1731-40.
7. K/DOQI. Clinical Practice Guidelines for Bone Metabolism and Disease in Chronic Kidney Disease (K/DOQI clinical practise guideline). American Journal of Kidney Diseases. 2003;42(4):s7-s201.
8. KDIGO. Clinical Practice Guideline for the Diagnosis, Evaluation, Prevention, and Treatment of Chronic Kidney Disease-Mineral and Bone Disorder (CKD-MBD). Kidney Int 2009;76(Supplement 113):S1-S130.
9. Navaneethan SD, Palmer SC, Craig JC, Elder GJ, Strippoli GF. Benefits and harms of phosphate binders in CKD: a systematic review of randomized controlled trials. Am J Kidney Dis. 2009;54(4):619-37.
10. Marks J, Debnam ES, Unwin RJ. Phosphate homeostasis and the renal-gastrointestinal axis. American Journal of Physiology - Renal Physiology. 2010;299(2):F285-F96.
11. Suyama T, Okada S, Ishijima T, lida K, Abe K, Nakai Y. High phosphorus diet-induced changes in NaPi-IIb phosphate transporter expression in the rat kidney: DNA microarray analysis. PLoS One. 2012;7(1):e29483.
12. Katai K, Tanaka H, Tatsumi S, Fukunaga Y, Genjida K, Morita K, et al. Nicotinamide inhibits sodium-dependent phosphate cotransport activity in rat small intestine. Nephrol Dial Transplant. 1999;14(5):1195-201.
13. Giral H, Caldas Y, Sutherland E, Wilson P, Breusegem S, Barry N, et al. Regulation of rat intestinal Na-dependent phosphate transporters by dietary phosphate. Am J Physiol Renal Physiol. 2009;297(5):F1466-F75.
14. Sabbagh Y, Giral H, Caldas Y, Levi M, Schiavi SC. Intestinal phosphate transport. Adv Chronic Kidney Dis. 2011;18(2):85-90.
15. Gattineni J, Bates C, Twombley K, Dwarakanath V, Robinson ML, Goetz R, et al. FGF23 decreases renal NaPi-2a and NaPi-2c expression and induces hypophosphatemia in vivo predominantly via FGF receptor 1. Am J Physiol Renal Physiol. 2009;297(2):F282-F91.
16. Hattenhauer O, Traebert M, Murer H, Biber J. Regulation of small intestinal Na-P(i) type IIb cotransporter by dietary phosphate intake. Am J Physiol. 1999;277(4 Pt 1):G756-G62.
17. Xu H, Bai L, Collins JF, Ghishan FK. Age-dependent regulation of rat intestinal type IIb sodium-phosphate cotransporter by 1,25-(OH)2 vitamin D3. AJP - Cell Physiology. 2002;282(3):C487-C93.
18. Eto N, Miyata Y, Ohno H, Yamashita T. Nicotinamide prevents the development of hyperphosphataemia by suppressing intestinal sodium-dependent phosphate transporter in rats with adenine-induced renal failure. Nephrol Dial Transplant. 2005;20(7): 1378-84.
19. Takahashi Y, Tanaka A, Nakamura T, Fukuwatari T, Shibata K, Shimada N, et al. Nicotinamide suppresses hyperphosphatemia in hemodialysis patients. Kidney Int. 2004;65(3):1099-104.
20. Rahmouni K, Araar B, Harbouche L, Shahapuni I, Esper NE, Fournier A. Use of nicotinamide as phosphate binder could reduce by half the cost of hyperphosphatemia control by Sevelamer in dialysis patients. The 38th Annual Meeting of the American Society of Nephrology. 2005:SP240.
21. Cheng SC, Young DO, Huang Y, Delmez JA, Coyne DW. A Randomized, Double-Blind, Placebo-Controlled Trial of Niacinamide for Reduction of Phosphorus in Hemodialysis Patients. Clin J Am Soc Nephrol. 2008;3(4):1131-8.
22. Young DO, Cheng SC, Delmez JA, Coyne DW. The effect of oral niacinamide on plasma phosphorus levels in peritoneal dialysis patients. Perit Dial Int. 2009;29(5):562-7.
23. Mikolasevic I, Zutelija M, Mavrinac V, Orlic L. Dyslipidemia in patients with chronic kidney disease: etiology and management. Int J Nephrol Renovasc Dis. 2017;10:35-45.
24. Baigent C, Landray MJ, Reith C, Emberson J, Wheeler DC, Tomson C, et al. The effects of lowering LDL cholesterol with simvastatin plus ezetimibe in patients with chronic kidney disease (Study of Heart and Renal Protection): a randomised placebo-controlled trial. Lancet. 2011;377(9784):2181-92.
25. März W, Genser B, Drechsler C, Krane V, Grammer TB, Ritz E, et al. Atorvastatin and low-density lipoprotein cholesterol in type 2 diabetes mellitus patients on hemodialysis. Clin J Am Soc Nephrol. 2011;6(6):1316-25.
26. Amann K. Media calcification and intima calcification are distinct entities in chronic kidney disease. Clin J Am Soc Nephrol. 2008;3(6):1599-605.
27. Haffner SM, Gruber KK, Aldrete G, Jr., Morales PA, Stern MP, Tuttle KR. Increased lipoprotein(a) concentrations in chronic renal failure. J Am Soc Nephrol. 1992;3(5):1156-62.
28. Trenkwalder E, Gruber A, Konig P, Dieplinger H, Kronenberg F. Increased plasma concentrations of LDL-unbound apo(a) in patients with end-stage renal disease. Kidney Int. 1997;52(6):1685-92.
29. Kotani K, Banach M. Lipoprotein(a) and inhibitors of proprotein convertase subtilisin/kexin type 9. J Thorac Dis. 2017;9(1):E78-E82.
30. Carlson LA, Hamsten A, Asplund A. Pronounced lowering of serum levels of lipoprotein Lp(a) in hyperlipidaemic subjects treated with nicotinic acid. J Intern Med. 1989;226(4):271-6.
31. Chennamsetty I, Kostner KM, Claudel T, Vinod M, Frank S, Weiss TS, et al. Nicotinic acid inhibits hepatic APOA gene expression: studies in humans and in transgenic mice. J Lipid Res. 2012;53(11):2405-12.
32. Digby JE, Ruparelia N, Choudhury RP. Niacin in cardiovascular disease: recent preclinical and clinical developments. Arterioscler Thromb Vasc Biol. 2012;32(3):582-8.
33. Tunaru S, Lattig J, Kero J, Krause G, Offermanns S. Characterization of determinants of ligand binding to the nicotinic acid receptor GPR109A (HM74A/PUMA-G). Mol Pharmacol. 2005;68(5):1271-80.
34. Gouni-Berthold I, Berthold HK. The role of niacin in lipid-lowering treatment: are we aiming too high? Curr Pharm Des. 2013;19(17):3094-106.
35. Medice Arzneimittel Putter GmbH & Co.KG. A single-center, randomized, open-label, single-dose, 2 treatment, 4 period, two-stage, cross-over bioavailability study with nicotinamide 250 mg modified release capsules versus a reference formulation (Nicotinsäureamid 200 mg JENAPHARM®) in fasting and fed healthy subjects. 2015 Medice Study No. 6520-9961-06, EudraCT No. 2013-002923-41.
36. Medice Arzneimittel Putter GmbH & Co.KG. Dose-Finding Trial of Nicotinamide in Dialysis-Dependent Patients with Hyperphosphataemia, The DONATO Trial. 2012 Medice Study No. 6520-9961-03, EudraCT No. 2009-015821-34.
37. Yang J, Adams JD. Nicotinamide and its pharmacological Properties for Clinical Therapy. Drug Design Reviews online. 2004;1:4.
38. Mogielnicki A, Kramkowski K, Pietrzak L, Buczko W. N-methylnicotinamide inhibits arterial thrombosis in hypertensive rats. J Physiol Pharmacol. 2007;58(3):515-27.
39. Schiavi SC, Tang W, Bracken C, O'Brien SP, Song W, Boulanger J, et al. Npt2b Deletion Attenuates Hyperphosphatemia Associated with CKD. J Am Soc Nephrol. 2012;23:11691-700.
40. Galeano C, Navarro P, Teruel JL, Ortuno J. [Treatment of hyperphosphatemia in dialyzed patients with nicotinamide]. Nefrologia. 2005;25(6):725-6.
41. Rottembourg JB, Launay-Vacher V, Massard J. Thrombocytopenia induced by nicotinamide in hemodialysis patients. Kidney Int. 2005;68(6):2911-2.
42. Delanaye P, Weekers L, Krzesinski JM. Diarrhea induced by high doses of nicotinamide in dialysis patients. Kidney Int. 2006;69(10):1914.
43. Olivero J, Garney P, Armentrout B, Mays L, Holland R. Nicotinamide Used for the treatment of Hyperhosphatemia in Hemodialysis Patients. The 39th Annual Meeting of the American Society of Nephrology, November 14-19,. 2006:F-PO104.
44. del Carmen Reinoso S, Grinblat N, Santos JC, al. e. Nicotinamide: an Alternative for Hyperphosphatemia in Dialysis Patients. World Congress of Nephrology. 2009:Su570.
45. Shahbazian H, Zafar MA, Ghorbani A, Abbaspour MR, Belladi Musavi SS, Hayati F, et al. Oral nicotinamide reduces serum phosphorus, increases HDL, and induces thrombocytopenia in hemodialysis patients: a double-blind randomized clinical trial. Nefrologia. 2011;31(1):58-65.
46. Vasantha J, Soundararajan P, Vanitharani N, Kannan G, Thennarasu P, Neenu G, et al. Safety and efficacy of nicotinamide in the management of hyperphosphatemia in patients on hemodialysis. Indian J Nephrol. 2011;21(4):245-9.
47. Allam S, El-Hamamsy M, El Sharkawy. The Effect of Coadminstration of Nicotinamide and Calcium-based Phosphate Binder on Hyperphophatemia in Patients Undergoing Hemodialysis. Advances in Natural Science. 2012;5(1):1-9.
48. Lenglet A, Liabeuf S, el EN, Brisset S, Mansour J, Lemaire-Hurtel AS, et al. Efficacy and safety of nicotinamide in haemodialysis patients: the NICOREN study. Nephrol Dial Transplant. 2016, Pubmed ID 27190329 (2016 May 4).
49. El Borolossy R, El Wakeel LM, El Hakim I, Sabri N. Efficacy and safety of nicotinamide in the management of hyperphosphatemia in pediatric patients on regular hemodialysis. Pediatr Nephrol. 2016;31(2):289-96.
50. Sampathkumar K, Selvam M, Sooraj YS, Gowthaman S, Ajeshkumar RN. Extended release nicotinic Acid - a novel oral agent for phosphate control. Int Urol Nephrol. 2006;38(1):171-4.
51. Gillmor HA, Bolton CH, Hopton M, Moore WP, Perrett D, Bingley PJ, et al. Measurement of nicotinamide and N-methyl-2-pyridone-5-carboxamide in plasma by high performance liquid chromatography. Biomed Chromatogr. 1999;13(5):360-2.
52. Petley A, Macklin B, Renwick AG, Wilkin TJ. The pharmacokinetics of nicotinamide in humans and rodents. Diabetes. 1995;44(2):152-5.
53. N.N. Niacinamide. Monograph. Altern Med Rev. 2002;7(6):525-9.
54. European Commission, Health & Consumer Protection Directorate-General. Opinion of the Scientific Committee on Food on the Tolerable Upper Intake Level of Nicotinic Acid and Nicotinamide (Niacin). SCF/CS/NUT/UPPLEV/39 Final Report, 6th May 2002.
55. Neradova A, Schumacher S, Lux P, et al. Phosphate binders and vitamin K absorption. 53rd ERA-EDTA Congress, Vienna 2016; Poster MP377
56. Forster IC, Hernando N, Biber J, Murer H. Phosphate transporters of the SLC20 and SLC34 families. Mol Aspects Med. 2013; 34 (2-3):386-95.
57. Pulskens WP, Verkaik M, Sheedfar F, van Loon EP, van de Sluis B, Vervloet MG, et al. Deregulated Renal Calcium and Phosphate Transport during Experimental Kidney Failure. PLoS One. 2015; 10 (11):e0142510.
58. Ketteler M. Phosphate Metabolism in CKD Stages 3-5: Dietary and Pharmacological Control. Int J Nephrol. 2011; 2011:970245.
59. Sprague SM, Abboud H, Qiu P, Dauphin M, Zhang P, Finn W. Lanthanum carbonate reduces phosphorus burden in patients with CKD stages 3 and 4: a randomized trial. Clin J Am Soc Nephrol. 2009; 4 (1):178-85.
60. Oliveira RB, Cancela AL, Graciolli FG, dos Reis LM, Draibe SA, Cuppari L, et al. Early control of PTH and FGF23 in normophosphatemic CKD patients: a new target in CKD-MBD therapy? Clin J Am Soc Nephrol. 2010; 5 (2):286-91.
61. Block GA, Ix JH, Ketteler M, Martin KJ, Thadhani RI, Tonelli M, et al. Phosphate Homeostasis in CKD: Report of a Scientific Symposium Sponsored by the National Kidney Foundation. Am J Kidney Dis. 2013; 62 (3):457-73.
62. Gutierrez O, Isakova T, Rhee E, Shah A, Holmes J, Collerone G, et al. Fibroblast growth factor-23 mitigates hyperphosphatemia but accentuates calcitriol deficiency in chronic kidney disease. J Am Soc Nephrol. 2005; 16 (7):2205-15.
63. Marks J, Srai SK, Biber J, Murer H, Unwin RJ, Debnam ES. Intestinal phosphate absorption and the effect of vitamin D: a comparison of rats with mice. Exp Physiol. 2006; 91 (3):531-7.
64. Go AS, Chertow GM, Fan D, McCulloch CE, Hsu CY. Chronic kidney disease and the risks of death, cardiovascular events, and hospitalization. N Engl J Med. 2004; 351 (13): 1296-305.
65. Kestenbaum B, Sampson JN, Rudser KD, Patterson DJ, Seliger SL, Young B, et al. Serum phosphate levels and mortality risk among people with chronic kidney disease. J Am Soc Nephrol. 2005; 16 (2):520-8.
66. Block GA, Klassen PS, Lazarus JM, Ofsthun N, Lowrie EG, Chertow GM. Mineral metabolism, mortality, and morbidity in maintenance hemodialysis. J Am Soc Nephrol. 2004; 15 (8):2208-18.
67. Frei U, Schober-Halstenberg H-J. (2008). Nierenersatztherapie in Deutschland Bericht über Dialysebehandlung und Nierentransplantation in Deutschland 2006-2007. Berlin: QuaSi-Niere GmbH
68. Foley RN, Parfrey PS, Sarnak MJ. Clinical epidemiology of cardiovascular disease in chronic renal disease. Am J Kidney Dis. 1998; 32 (5 Suppl 3):S112-S9.
69. Moe SM, Chen NX. Pathophysiology of vascular calcification in chronic kidney disease. Circ Res. 2004; 95 (6):560-7.
70. Konishi H, Miyauchi K, Tsuboi S, Ogita M, Naito R, Dohi T, et al. Plasma lipoprotein(a) predicts major cardiovascular events in patients with chronic kidney disease who undergo percutaneous coronary intervention. Int J Cardiol. 2016; 205:50-3.
71. Yun JS, Ahn YB, Song KH, Yoo KD, Park YM, Kim HW, et al. Lipoprotein(a) predicts a new onset of chronic kidney disease in people with Type 2 diabetes mellitus. Diabet Med. 2016; 33 (5):639-43.
72. Kostner KM, Marz W, Kostner GM. When should we measure lipoprotein (a)? Eur Heart J. 2013; 34 (42):3268-76.
73. Remington, The Science and Practice of Pharmacy", 22nd edition, 2013, pp. 777 - 1070
74. Remington, The Science and Practice of Pharmacy, 22nd Edition, 2012, volume 1: "The Science of Pharmacy", pages 1049-1070
75. Remington, The Science and Practice of Pharmacy", 22nd edition, 2013, pp. 981, 982, 989 -998

## Claims

1. A pharmaceutical preparation comprising a pharmaceutically effective amount of modified release nicotinamide for use in a method of preventing and/or treating of elevated serum phosphate levels (hyperphosphatemia) and/or dyslipidemia, particularly dysregulation of lipid metabolism, particularly elevation of serum Lipoprotein(a) (Lp(a)) levels, both particularly resulting from renal failure, wherein about 25 - 55 % by weight of the nicotinamide is released from the pharmaceutical preparation at a pH of about 0.5 to about 1.5 at a time period of about 2 hours.

2. The pharmaceutical preparation for use in a method of claim 1, wherein said hyperphosphatemia and/or dyslipidemia results from chronic kidney failure, from of end-stage renal disease, and/or from hemodialysis.

3. The pharmaceutical preparation for use in a method of claim 1 or claim 2, **wherein it is administered** parenterally or orally.

4. The pharmaceutical preparation for use in a method of one or more of the preceding claims, wherein the nicotinamide is to be administered in unit doses up to about 2000 mg per day, preferably in unit doses ranging from about 250 to about 2000 mg per day.

5. The pharmaceutical preparation for use in a method of one or more of the preceding claims, wherein the nicotinamide is to be administered before, with and/or after meals and/or before going to bed, independently from food intake and before and/or after hemodialysis or peritoneal dialysis treatment.

6. The pharmaceutical preparation for use in a method of one or more of the preceding claims, wherein the pharmaceutical preparation comprising a pharmaceutically effective amount of modified release nicotinamide is administered once or twice daily independently from food intake, preferably once daily, further preferably before going to bed.

7. The pharmaceutical preparation for use in a method of one or more of the preceding claims, wherein further at least one phosphate binder is administered.

8. The pharmaceutical preparation for use in a method of claim 7, wherein the pharmaceutical preparation comprising a pharmaceutically effective amount of modified release nicotinamide is administered at a time different from the administration of the at least one phosphate binder, preferably with a time difference of at least one hour, further preferably at least two hours, even further preferably at least three hours.

9. The pharmaceutical preparation for use in a method of claim 7 or 8, wherein the at least one phosphate binder is not sevelamer and/or a derivative thereof, preferably wherein the at least one phosphate binder is calcium acetate, calcium carbonate and/or lanthanum carbonate and/or an aluminum containing phosphate binder and/or a phosphate binder containing iron.

10. A pharmaceutical preparation comprising modified release nicotinamide, wherein about 25 - 55 % by weight of the nicotinamide is released from the pharmaceutical preparation at a pH of about 0.5 to about 1.5 at a time of about 2 hours.

11. The pharmaceutical preparation according to claim 10, comprising a formulation comprising nicotinamide covered with a modified release coating.

12. The pharmaceutical preparation according to claim 11, wherein the modified release coating comprises at least one binder and at least one modified release agent, preferably wherein the modified release agent comprises ethyl cellulose and hydroxypropyl methylcellulose.

13. The pharmaceutical preparation according to claim 11 or 12, wherein the modified release coating comprises ethyl cellulose and hydroxypropyl methylcellulose in a weight ratio of about 10: to about 20:1, preferably about 12: 1 to about 16:1.

14. The pharmaceutical preparation according to one or more of claims 10 to 13, wherein the formulation comprising nicotinamide is in the form of pellets.

15. The pharmaceutical preparation according to one or more of claims 10 to 14, wherein the pharmaceutical preparation is in the form of a capsule comprising pellets of modified release nicotinamide.

16. The pharmaceutical preparation according to one or more of claims 10 to 15, wherein the pharmaceutical preparation comprises from about 100 mg to about 1500 mg of nicotinamide.

17. A kit-of-parts, comprising the pharmaceutical preparation according to one or more of claims 10 to 16 and at least one phosphate binder.
